(19) Europäisches Patentamt
European Patent Office
Office européen des brevets

(11) **EP 4 119 650 A1**

(12) **EUROPEAN PATENT APPLICATION**
published in accordance with Art. 153(4) EPC

(43) Date of publication:
**18.01.2023 Bulletin 2023/03**

(21) Application number: **21768043.8**

(22) Date of filing: **09.03.2021**

(51) International Patent Classification (IPC):
**C12M 1/00** (2006.01)    **C12M 1/26** (2006.01)
**C12N 15/87** (2006.01)    **C12Q 1/24** (2006.01)

(52) Cooperative Patent Classification (CPC):
**C12M 1/00; C12M 1/26; C12N 15/87; C12Q 1/24**

(86) International application number:
**PCT/JP2021/009347**

(87) International publication number:
**WO 2021/182479 (16.09.2021 Gazette 2021/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.03.2020 JP 2020041164**

(71) Applicant: **Hynts Tech Corporation**
**Kitakyushu-shi, Fukuoka 808-0135 (JP)**

(72) Inventor: **MIYAKE, Takeo**
**Tokyo 169-8050 (JP)**

(74) Representative: **Engelhard, Markus**
**Boehmert & Boehmert**
**Anwaltspartnerschaft mbB**
**Pettenkoferstrasse 22**
**80336 München (DE)**

(54) **INTRACELLULAR SUBSTANCE INTRODUCTION AND EXTRACTION TECHNIQUE VIA COMPOSITE NANOTUBES**

(57) The present invention delivers a substance such as a reagent into a cell. A tubular body coated with an electroconductive polymer for introducing a substance into a cell and/or recovering a substance from a cell. The use of a tubular body coated with an electroconductive polymer to introduce a substance into a cell and/or recover a substance from a cell. A device for introducing a substance into a cell and/or recovering a substance from a cell, the device being equipped with a substrate and a tubular body coated with an electroconductive polymer, and the tubular body being positioned on the substrate. A system for introducing a substance into a cell and/or recovering a substance from a cell, the system being equipped with the abovementioned device, a voltage supply unit for supplying a voltage, and, as needed, electrodes.

**Description**

[Technical Field]

The present invention relates to a technology for controllably moving a substance into/from a cell.

[Background Art]

Technologies for delivering a substance such as a reagent into a cell by using a nanotube structure such as a nanoneedle or nanostraw have been known. However, such technologies materialize introduction and recovery of a substance through puncturing a hole in the cell membrane by applying a high voltage (about 0.1 to 10 V) ("electroporation"). However, application of a high voltage results in undesirable changes in cellular function such as cell death or oncogenesis.

[Citation List]

[Patent Literature]

[PTL 1] Japanese National Phase PCT Laid-open Publication No. 2019-528782
[PTL 2] Japanese National Phase PCT Laid-open Publication No. 2019-517273

[Non Patent Literature]

[NPL 1] JJ Van Dersarl, AM Xu, NA Melos, Nanostraws for direct fluidic intracellular access, Nano letters 12 (8), 3881-3886, 2011.
[NPL 2] A Tay, N Melosh, Nanostructured Materials for Intracellular Cargo Delivery, Accounts of chemical research 52 (9), 2462-2471, 2019.
[NPL 3] Y Cao, H Chen, R Qiu, M Hanna, E Ma, M Hjort, A Zhang, RS Lewis, JC Wu, NA Melosh, Universal intracellular biomolecule delivery with precise dosage control, Science advances 4 (10), eaat8131, 2019.
[NPL 4] Rui Wen, Aihua Zhang, Di Liu, Jianming Feng, Jiang Yang, Dehua Xia, Ji Wang, Chunwei Li, Tao Zhang, Ning Hu, Tian Hang, Gen He, Xi Xie, Intracellular Delivery and Sensing System Based on Electroplated Conductive Nanostraw Arrays, ACS applied materials & interfaces, doi.org/10.1021/acsami.9b15619, 2019.
[NPL 5] Gen He, Jianming Feng, Aihua Zhang, Lingfei Zhou, Rui Wen, Jiangming Wu, Chengduan Yang, Jiang Yang, Chunwei Li, Demeng Chen, Ji Wang, Ning Hu, Xi Xie, Multifunctional Branched Nanostraw-Electroporation Platform for Intracellular Regulation and Monitoring of Circulating Tumor Cells, Nano letters 19 (10), 7201-7209, 2019.

[Summary of Invention]

[Solution to Problem]

For example, the present disclosure provides the following items.

(Item 1)

A tubular body coated with a conductive macromolecule for use in introducing a substance into a cell and/or recovering a substance from a cell.

(Item 2)

Use of a tubular body coated with a conductive macromolecule for introducing a substance into a cell and/or recovering a substance from a cell.

(Item 3)

The tubular body or use of item 1 or 2, wherein the tubular body is made of a conductor.

(Item 4)

The tubular body or use of item 3, wherein the conductor comprises a metal.

(Item 5)

The tubular body or use of item 4, wherein the metal comprises a metal that can be applied by electroless plating.

(Item 6)

The tubular body or use of item 4 or 5, wherein the metal comprises at least one metal selected from the group consisting of gold, platinum, silver, nickel, and alloys thereof.

(Item 7)

The tubular body or use of any one of items 1 to 4, wherein the conductive macromolecule has a property of expanding and contracting when a voltage is applied.

(Item 8)

The tubular body or use of item 7, wherein an absolute value of the voltage is 500 mV or less.

(Item 9)

The tubular body or use of any one of items 7 to 8, wherein an absolute value of the voltage is 100 mV or less.

(Item 10)

The tubular body or use of any one of items 7 to 9, wherein an absolute value of the voltage is 50 mV or less.

(Item 11)

The tubular body or use of any one of items 7 to 10, wherein the voltage is an AC voltage.

(Item 12)

The tubular body or use of any one of items 1 to 11, wherein the conductive macromolecule comprises at least one macromolecule selected from the group consisting of poly(3,4-ethylene dioxythiophene) (PEDOT), polythiophene, polyacetylene, polyaniline, polypyrrole, and combinations thereof.

(Item 13)

The tubular body or use of any one of items 1 to 12, wherein the tubular body has an inner diameter of 2 $\mu$m or less.

(Item 14)

The tubular body or use of any one of items 1 to 13, wherein the tubular body has an outer diameter of 4 $\mu$m or less.

(Item 15)

The tubular body or use of any one of items 1 to 14, wherein the tubular body has a length of 5 $\mu$m or greater and 50 $\mu$m or less.

(Item 16)

The tubular body or use of any one of items 1 to 15, wherein the substance has an arithmetic mean diameter of 1 nm or greater and 2 $\mu$m or less.

(Item 17)

The tubular body or use of any one of items 1 to 16, wherein the substance comprises at least one selected from the group consisting of a low molecular weight compound, a nucleic acid, a peptide, a protein, and an organelle.

(Item 18)

A device for use in introducing a substance into a cell and/or recovering a substance from a cell, comprising a tubular body coated with a conductive macromolecule, a substrate, and an electrode, wherein the tubular body is disposed on the substrate.

(Item 19)

The device of item 18, wherein the device comprises a plurality of the tubular bodies.

(Item 20)

The device of item 18 or 19, wherein the device further comprises a storage unit for storing the substance.

(Item 21)

The device of any one of items 18 to 20, wherein the substrate comprises polycarbonate and/or polyethylene terephthalate.

(Item 22)

The device of any one of items 20 to 21, wherein the storage unit has a structure, which has an inside that is a void, is directly connected to the substrate, and has a tube through which an electrode can be inserted into the void.

(Item 23)

The device of any one of items 18 to 22, wherein the storage unit is comprised of at least one material selected from the group consisting of glass, ceramic, macromolecule, and metal.

(Item 24)

The device of any one of items 18 to 23, wherein the tubular body further comprises a feature of any one of items 1 to 17.

(Item 25)

The device of any one of items 20 to 24, wherein the storage unit comprises the substance.

(Item 26)

The device of any one of items 18 to 25, wherein the electrode is provided with two poles for applying a voltage to the conductive macromolecule and a storage unit.

(Item 27)

A system for use in introducing a substance into a cell and/or recovering a substance from a cell, comprising:

the device of any one of items 18 to 26; and

a voltage supplying unit for supplying a voltage.

(Item 28)

The system of item 27, further comprising a container comprising the cell.

(Item 29)

The system of item 27 or 28, further comprising an observation unit for differential interference contrast observation of the cell in a container from below.

(Item 30)

The system of item 29, wherein the observation unit is materialized by a differential interference contrast prism.

(Item 31)

The system of any one of items 27 to 30, further comprising a display unit for displaying an image from the observation unit.

(Item 32)

A method for introducing a substance into a cell and/or recovering a substance from a cell, comprising the steps of:

    (a) providing the device of any one of items 18 to 26 or the system of any one of items 27 to 31; and
    (b) applying a voltage to the tubular body over a desired period of time.

(Item 33)

A method of fabricating a device comprising a tubular body coated with a conductive macromolecule and a substrate, comprising the step of:
securing a membrane of the conductive macromolecule to a substrate on which the tubular body is disposed at one or more points to coat the tubular body.

(Item 34)

The method of item 33, wherein the step of coating comprises the step of securing the membrane of the conductive macromolecule at two or more points to coat the tubular body.

(Item 35)

The method of item 33 or 34, wherein the step of coating is achieved by providing working electrodes that contact the substrate at two or more points.

(Item 36)

The method of any one of items 33 to 35, wherein the substrate on which the tubular body is disposed is fabricated by a process comprising the step of electroless plating of a metal on track-etched polycarbonate and the step of etching a metal plated surface.

(Item 37)

The method of any one of items 33 to 36, wherein an inner diameter of the tubular body is 0.01 $\mu$m to 2 $\mu$m, and an outer diameter is 0.1 $\mu$m to 5 $\mu$m.

(Item 1A)

A tubular body coated with a conductive macromolecule for introducing a substance into a cell and/or recovering a substance from a cell.

(Item 2A)

Use of a tubular body coated with a conductive macromolecule for introducing a substance into a cell and/or recovering a substance from a cell.

(Item 3A)

The tubular body or use of item 1A or 2A, wherein the tubular body is made of a conductor.

(Item 4A)

The tubular body or use of item 3A, wherein the conductor comprises a metal.

(Item 5A)

The tubular body or use of item 4A, wherein the metal comprises a metal that can be applied by electroless plating.

(Item 6A)

The tubular body or use of item 4A or 5A, wherein the metal comprises at least one metal selected from the group consisting of gold, platinum, silver, nickel, and alloys thereof.

(Item 7A)

The tubular body or use of any one of items 1A to 4A, wherein the conductive macromolecule has a property of expanding and contracting when a voltage is applied.

(Item 8A)

The tubular body or use of item 7A, wherein an absolute value of the voltage is 500 mV or less.

(Item 9A)

The tubular body or use of any one of items 7A to 8A, wherein an absolute value of the voltage is 100 mV or less.

(Item 10A)

The tubular body or use of any one of items 7A to 9A, wherein an absolute value of the voltage is 50 mV or less.

(Item 11A)

The tubular body or use of any one of items 7A to 10A, wherein the voltage is an AC voltage.

(Item 12A)

The tubular body or use of any one of items 1A to 11A, wherein the conductive macromolecule comprises at least one macromolecule selected from the group consisting of poly(3,4-ethylene dioxythiophene) (PEDOT), polythiophene, polyacetylene, polyaniline, polypyrrole, and combinations thereof.

(Item 13A)

The tubular body or use of any one of items 1A to 12A, wherein the tubular body has an inner diameter of 2 μm or less.

(Item 14A)

The tubular body or use of any one of items 1A to 13A, wherein the tubular body has an outer diameter of 4 μm or less.

(Item 15A)

The tubular body or use of any one of items 1A to 14A, wherein the tubular body has a length of 5 μm or greater and 50 μm or less.

(Item 16A)

The tubular body or use of any one of items 1A to 15A, wherein the substance has an arithmetic mean diameter of 1 nm or greater and 2 μm or less.

(Item 17A)

The tubular body or use of any one of items 1A to 16A, wherein the substance comprises at least one selected from the group consisting of a low molecular weight compound, a nucleic acid, a peptide, a protein, and an organelle.

(Item 18A)

A device for introducing a substance into a cell and/or recovering a substance from a cell, comprising a tubular body coated with a conductive macromolecule, a substrate, and an electrode, wherein the tubular body is disposed on the substrate.

(Item 19A)

The device of item 18A, wherein the device comprises a plurality of the tubular bodies.

(Item 20A)

The device of item 18A or 19A, wherein the device further comprises a storage unit for storing the substance.

(Item 21A)

The device of any one of items 18A to 20A, wherein the substrate comprises polycarbonate and/or polyethylene terephthalate.

(Item 22A)

The device of any one of items 20A to 21A, wherein the storage unit has a structure, which has an inside that is a void, is directly connected to the substrate, and has a tube through which an electrode can be inserted into the void.

(Item 23A)

The device of any one of items 18A to 22A, wherein the storage unit is comprised of at least one material selected from the group consisting of glass, ceramic, macromolecule, and metal.

(Item 24A)

The device of any one of items 18A to 23A, wherein the tubular body further comprises a feature of any one of items 1A to 17A.

(Item 25A)

The device of any one of items 20A to 24A, wherein the storage unit comprises the substance.

(Item 26A)

The device of any one of items 18A to 25A, wherein the electrode is provided with two poles for applying a voltage to the conductive macromolecule and a storage unit.

(Item 27A)

A system for introducing a substance into a cell and/or recovering a substance from a cell, comprising:

the device of any one of items 18A to 26A; and
a voltage supplying unit for supplying a voltage.

(Item 28A)

The system of item 27A, further comprising a container comprising the cell.

(Item 29A)

The system of item 27A or 28A, further comprising an observation unit for differential interference contrast observation of the cell in a container from below.

(Item 30A)

The system of item 29A, wherein the observation unit is materialized by a differential interference contrast prism.

(Item 31A)

The system of any one of items 27A to 30A, further comprising a display unit for displaying an image from the observation unit.

(Item 32A)

The system of any one of items 27A to 31A, wherein insertion of a tubular body contained in the device into the cell can be adjusted in units of 0.1 $\mu$m.

(Item 33A)

A method for introducing a substance into a cell and/or recovering a substance from a cell, comprising the steps of:

(a) providing the device of any one of items 18A to 26A or the system of any one of items 27A to 32A; and
(b) applying a voltage to the tubular body over a desired period of time.

(Item 34A)

A method of fabricating a device comprising a tubular body coated with a conductive macromolecule and a substrate, comprising the step of:
securing a membrane of the conductive macromolecule to a substrate on which the tubular body is disposed at one or more points to coat the tubular body.

(Item 35A)

The method of item 34A, wherein the step of coating comprises the step of securing the membrane of the conductive macromolecule at two or more points to coat the tubular body.

(Item 36A)

The method of item 34A or 35A, wherein the step of coating is achieved by providing working electrodes that contact the substrate at two or more points.

(Item 37A)

The method of any one of items 34A to 36A, wherein the substrate on which the tubular body is disposed is fabricated by a process comprising the step of electroless plating of a metal on track-etched polycarbonate and the step of etching a metal plated surface.

(Item 38A)

The method of any one of items 34A to 37A, wherein an inner diameter of the tubular body is 0.01 $\mu$m to 2 $\mu$m, and an outer diameter is 0.1 $\mu$m to 5 $\mu$m.

(Item 39A)

A method of activating a cell, comprising the step of introducing a mitochondrion into the cell by using the device of any one of items 18A to 26A or the system of any one of items 27A to 32A.

(Item 40A)

A method of controlling a cellular function, comprising the step of introducing a gene product (protein) into the cell by using the device of any one of items 18A to 26A or the system of any one of items 27A to 32A.

(Item 41A)

The method of item 40A, wherein the gene product comprises a gene product of Oct4.

(Item 42A)

A tubular body coated with a conductive macromolecule for use in introducing a substance into a cell and/or recovering a substance from a cell, wherein the conductive macromolecule comprises kinesin.

(Item 43A)

Use of a tubular body coated with a conductive macromolecule comprising kinesin for introducing a substance into a cell and/or recovering a substance from a cell.

(Item 44A)

A device for use in introducing a substance into a cell and/or recovering a substance from a cell, comprising a tubular body coated with a conductive macromolecule comprising kinesin, a substrate, and an electrode, wherein the tubular body is disposed on the substrate.

(Item 45A)

A system for use in introducing a substance into a cell and/or recovering a substance from a cell, comprising:

the device of item 44A; and
a voltage supplying unit for supplying a voltage.

(Item 46A)

A method for introducing a substance into a cell and/or recovering a substance from a cell, comprising the steps of:

(a) providing the device of item 44A or the system of item 45A;
(b) providing a microtube with a substance attached thereto; and
(c) applying a voltage to the tubular body over a desired period of time.

(Item 47A)

A method of fabricating a tubular body coated with a conductive macromolecule comprising kinesin, comprising the step of attaching kinesin to the tubular body.

(Item 48A)

A stamping system for use in inserting a composite nanotube into a cell, comprising:

a stamp comprising a tubular body coated with a conductive macromolecule;
an electrode; and
a voltage supplying unit for supplying a voltage.

(Item 49A)

A penetration observation system for use in inserting a composite nanotube into a cell, comprising:

the device of any one of items 18A to 26A and 44A;
a voltage supplying unit for supplying a voltage; and
a microscope.

(Item 50A)

A composite nanotube thin membrane and stamp kit for delivering a substance into a cell or extracting a substance from a cell, comprising:

a composite nanotube thin membrane comprising a tubular body coated with a conductive macromolecule; and
a stamp.

(Item 51A)

A method of evaluating cell activity, comprising the step of:
measuring a quantity of a marker substance within a cell by using a tubular body coated with a conductive macromolecule.

(Item 52A)

The method of item 50A, further comprising the step of introducing a mitochondrion into a cell.

(Item 53A)

A method of introducing a cell using a microsphere, comprising the steps of:

(a) providing the device of any one of items 18A to 26A and 44A, or the system of any one of items 27A to 32A and 45A;
(b) providing a microsphere; and
(c) applying a voltage to the tubular body over a desired period of time.

(Item 54A)

The method of item 53A, further comprising the step of attaching a substance to the microsphere.

(Item 55A)

A method of extracting an intracellular substance, comprising the steps of:

(a) providing the device of any one of items 18A to 26A and 44A, or the system of any one of items 27A to 32A and 45A; and
(b) applying a voltage to the tubular body over a desired period of time.

(Item 56A)

A method of cell reprogramming, comprising the steps of:

(a) providing the device of any one of items 18A to 26A and 44A, or the system of any one of items 27A to 32A and 45A;
(b) providing a gene or a gene product; and
(c) applying a voltage to the tubular body over a desired period of time.

(Item 57A)

A method of introducing a functional protein into a cell to modify a phenotype of the cell, comprising the steps of:

(a) providing the device of any one of items 18A to 26A and 44A, or the system of any one of items 27A to 32A and 45A;
(b) providing a functional protein; and
(c) applying a voltage to the tubular body over a desired period of time.

Conventional technologies have problems including harm to cells in terms of puncturing a hole in the cell membrane with a high voltage. On the other hand, the present disclosure is significantly advantageous in that a substance can be transported or extracted, without or with minimal adverse action on cells, and has excellent safety in terms of the ability to use a minute voltage. An attempt to insert a composite nanotube into a cell and apply electricity to control transport was successful. The inventors succeeded in simultaneously incorporating a stamping system and an operation to make a hollow nanotube.
The present disclosure is advantageous in terms of an example of a composite nanotube from coating a metal nanotube with a conductive macromolecule membrane and the ability to control transport of a substance by turning the electricity to the composite nanotube ON/OFF.
A specific embodiment is useful in that a metal nanotube coated with a conductive macromolecule can be inserted into a cell to deliver a substance such as a gene or reagent into the cell. The embodiment is useful in that a substance can be introduced or recovered by applying a minute voltage ($\pm$ 0.1 V or less, a potential lower than the membrane potential). The embodiment is useful in that the amount of substance released can be controlled by turning an applied voltage On/Off.
The present disclosure is useful in providing a high level biological cell (smart cell). Such a cell can be used in manufacturing, food industry, environment, medicine, etc. Such a cell can be used in the manufacture of cultured food products, pharmaceutical products, or macromolecules or manufacture of cultured tissue, and is useful in the field of regenerative medicine or cosmetology. A plurality of substances can be safely introduced/extracted a plurality times into/from many cells to enable function control, substance manufacture, function analysis, or substance use. The present disclosure enables in cell NMR in a cellular environment. Cell senescence can be improved, and cells that have become cancerous can be killed. Mitochondria can also be introduced. The present disclose enables controlling the cellular function and direct induction into differentiated cells (direct reprogramming). The present disclosure is useful in pharmaceuticals, industrial products, seasoning, fragrance, etc.as smart cells.
The present invention is intended so that one or more of the features described above can be provided not only as the explicitly disclosed combinations, but also as other combinations thereof. Additional embodiments and advantages of the present invention are recognized by those skilled in the art by reading and understanding the following detailed description as needed.

[Advantageous Effects of Invention]

A substance such as a reagent can be delivered into a cell. Specifically, the following effects are contemplated.
With one embodiment of the present disclosure, a substance can be transported and recovered by inserting a metal nanotube coated with a conductive macromolecule and applying a minute voltage, and long-term insertion is also enabled. While a metal nanotube alone could not control the amount of substance released from applying a voltage and entailed a disadvantage in terms of cells dying with passage of time due to intracellular substances being released outside, but this was able to be remedied.
Currently, cellular function controlling technologies including iPS cells require that a substance (gene, reagent, etc.) is safely delivered into a cell and are utilized in a broad range of applications such as cell therapy and regenerative medicine. The technology of the present disclosure can be expected to have practical use in such cosmetic/medical fields.
In one embodiment, more can be achieved if substances to be introduced are increased. For example, iPS cells can be established from introducing a gene for creating iPS cells or introducing a protein. This can also be applied to regenerative medicine applications by introducing mitochondria into a cell sheet through combined use with another technology (isolated mitochondria, LUCA Science).

For known nanotubes, cells are punctured by electroporation that applies a high voltage, which can result in cell death or oncogenesis. Since the present invention can be inserted directly into cells for treatment that applies a minute voltage in a form that suppresses release of intracellular substances to the outside by coating with a macromolecule membrane, the problem of the application of a high voltage described above can be solved.

Development of a technology that has materialized controlled transport of a substance into a cell with a minute voltage and long-term insertion of a composite nanotube as described herein cannot be found elsewhere. Patent Literatures 1 to 2, Non Patent Literatures 1 to 3, as well as Non Patent Literatures 4 to 5 each report formation of a conductive macromolecule membrane and nanotube structure using a metal or metal oxide by using a track-etched membrane, but do not attempt to insert a composite nanotube into a cell and apply electricity to control transport. Conventional methods are completely different from the present invention, including the principle of transport, in terms of puncturing a hole in a cell membrane with a high voltage. The methods of the present disclosure are highly safe in terms of being able to use a minute voltage.

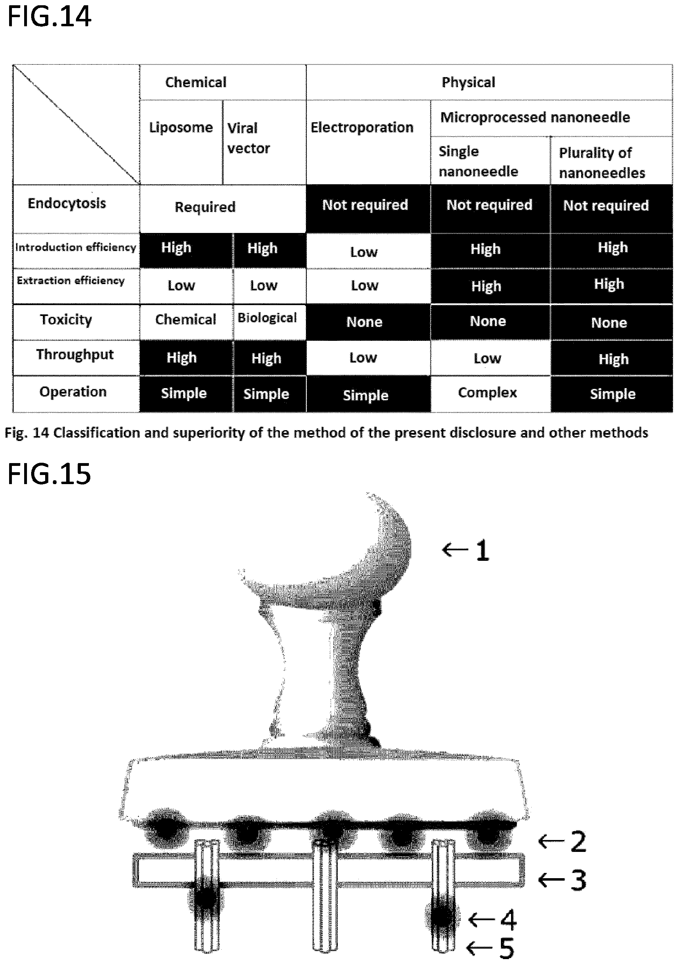

Currently, cellular function controlling technologies including iPS cells require that a substance (gene, reagent, etc.) is safely delivered into a cell and are utilized in a broad range of applications such as cell therapy and regenerative medicine. Technologies for introducing or extracting a substance into/from cells are roughly categorized into a chemical method and a physical method (Figure **14**). Conventional methods are similar to the present invention in terms of utilizing a nanoneedle, but have a disadvantage in terms of requiring the use of electroporation in combination. In comparison, the methods of the present invention have better features in every aspect and are expected to have a broad impact in the future.

If a substance can be delivered into cells safely by inserting needles together into a group of cells transported on a conveyer belt and applying electricity, a new cell manufacturing tool would be able to be provided. If any substance can be introduced safely into cells, use of the present invention in regenerative medicine/therapy for creating an organ, tissue, etc. from a cell unit as well as application of the present invention to the field of food products such as cultured cell meat or plant would be conceivable.

[Brief Description of Drawings]

[Figure 1] Figure **1** shows a system for penetrating a nanotube stamp into a cell, which incorporates DIC observation.

[Figure 2] Figure **2** is a diagram describing the substance introduction efficiency and cell viability rate with or without a DIC observation mechanism.

[Figure 3] Figure **3** shows the cell viability rate from intracellular insertion of a gold nanotube and composite nanotube.

[Figure 4] Figure **4** shows control of intracellular introduction of a fluorescent substance calcein by using a composite nanotube.

[Figure 5] Figure **5** shows the fluorescence intensity and frequency after introduction of calcein.

[Figure 6] Figure **6** shows control of intracellular introduction of a GFP protein by using a composite nanotube.

[Figure 7] Figure **7** shows composite nanotube mediated transport of isolated mitochondria.

[Figure 8] Figure **8** shows structural analysis of a PEDOT electropolymerized composite nanotube thin membrane.

[Figure 9] Figure **9** shows measurement of the concentration of calcein that has passed through a gold nanotube (0 min) and a composite nanotube thin membrane.

[Figure 10] Figure **10** shows the amount of calcein that passes through by turning the applied voltage On/Off.

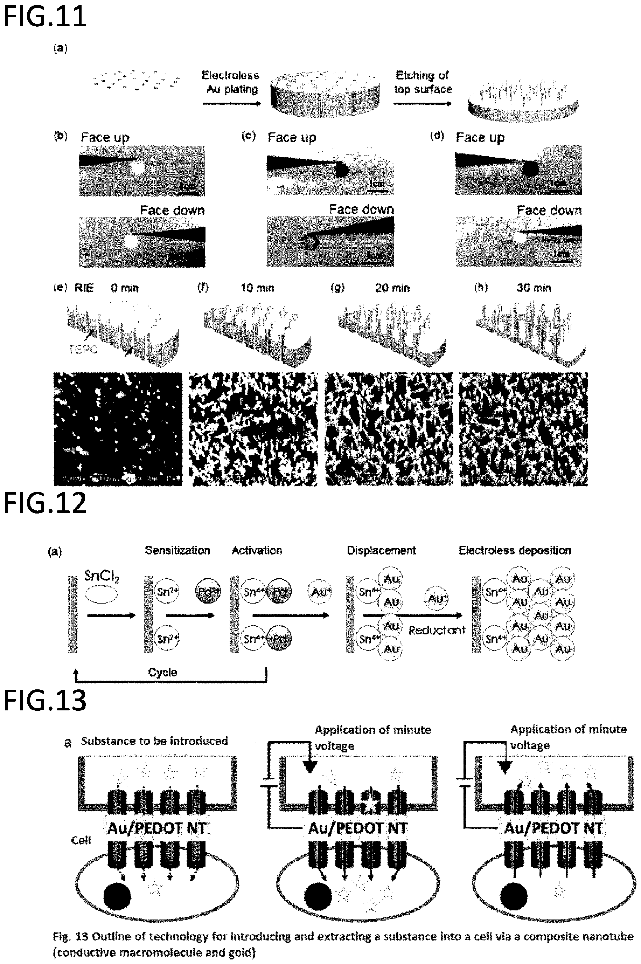

[Figure 11] Figure **11** shows fabrication of a nanostraw membrane. (a) Two steps of nanostraw fabrication: (1) electroless Au plating of track-etched polycarbonate (TEPC) membrane and (2) etching of top surface. (b to d) optical images of samples with an 8 mm diameter at different stages of nanostraw fabrication. TEPC membranes comprise size controlled pores. Pores with diameters of 0.4 $\mu$m, 0.6 $\mu$m, and 1.0 $\mu$m were used. (b) Images of the original TEPC membrane facing up and facing down. (c) Au is plated on the TEPC membrane surface and pore surface. (d) The top surface of an Au membrane was etched in two steps: (1) The Au surface is etched with aqua regia (comprising nitric acid and hydrochloric acid) and (2) The PC surface is etched with $O_2$RIE. (e to h) Au/TEPC nanostraw after RIE, 0 min (e), 10 min (f), 20 min (g), and 30 min (h).

[Figure 12] Figure **12** shows the process flow for fabricating an Au nanostraw on a polycarbonate membrane.

[Figure 13] Figure **13** shows an outline of technologies for introducing and extracting an intracellular substance via a composite nanotube (conductive macromolecule and gold).

[Figure 14] Figure **14** shows the classification and superiority of the method of the present disclosure and other methods.

[Figure 15] Figure **15** shows an exemplary stamp-shaped device of the present disclosure (stamping system).

[Figure 16] Figure **16** is a schematic diagram of the operation of an exemplary stamp-shaped device of the present disclosure.

[Figure 17] Figure **17** shows fluorescence images and intensity profiles of calcein stained cells.

[Figure 18] Figure **18** shows fluorescence images of viable cells and dead cells in an extraction area.

[Figure 19] Figure **19** shows the configuration of an experiment to introduce a microsphere into a cell by stamping.

[Figure 20] Figure **20** shows optical and fluorescence images after introducing a microsphere into a cell by a composite nanotube.

[Figure 21] Figure **21** shows a mechanism for intracellular transport of a substance using a biomolecular motor.

[Figure 22] Figure **22** shows transport of a biomolecular motor on a gold membrane and passage through a membrane via a nanotube.

[Figure 23] Figure **23** shows transport of a microtube with a labeled substance.

[Figure 24] Figure **24** shows introduction of an Oct4 protein into a cell.

[Figure 25] Figure **25** shows introduction of mitochondria into a cell.

[Figure 26] Figure **26** is a diagram describing the difference between settling rate and biomolecular motor rate.

[Description of Embodiments]

The present invention is described hereinafter in more detail. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

(Definitions)

The definitions of the terms and/or details of the basic technologies that are especially used herein are explained hereinafter as appropriate.

As used herein, "tubular body" refers to an elongated, bar-shaped, center portion-removed (hollow) body or entity, and is also referred to as an elongated hollow body or hollow tube. A tubular body is also expressed as a tube, straw, etc. The "outer diameter" and "inner diameter" of a tubular body are used in the meaning that is commonly used in the art. The diameter at the largest portion of a tubular body is referred to as the outer diameter, and the diameter of the hollow portion inside is referred to as the inner diameter. If the cross-section of a tubular body is a circle, the diameter is a diameter in the common meaning, but if not, the diameter refers to the arithmetic mean diameter when approximated as a circle. The inner diameter and outer diameter can be measured by any method that is known in the art. Since the target subjects are in the nano-scale, the subjects can be observed and measured with, for example, a scanning electron microscope. The length of a tubular body is used in the common meaning, and length can be measured by any method that is known in the art.

As used herein, a "cell" is defined in the broadest sense and refers to a small room-like substructure of all organisms. As used herein, a "cell" may be anything, as long as it is intended to move a substance, and preferably includes those encapsulating an organelle. Examples of cells include eukaryotes, prokaryotes, bacterial cells, fungal cells, etc.

As used herein, a "substance" is defined in the broadest sense and refers to any entity constituted by molecules or atoms. Examples thereof include low molecular weight compounds (typical examples include, but are not limited to, compounds with a molecular weight of 500 or less), middle molecular weight compounds (typical examples include, but are not limited to, compounds with a molecular weight of 500 to 10000), high molecular weight compounds (typical examples include, but are not limited to, compounds with a molecular weight of 10000 or greater), nucleic acids, peptides, proteins, organelles, etc. In the context of a subject of introduction or recovery (or retrieval) herein, a substance can be defined as a target that can be incorporated into a cell. As used herein, the diameter of a substance is expressed as an arithmetic mean diameter, and volume mean diameter is typically used. Calculation methods of such mean diameters are known in the art.

As used herein, "introduction" of a substance into a cell is defined in the broadest sense and refers to moving any substance from outside a cell to inside a cell.

As used herein, "recovery" or "retrieval" of a substance from a cell are interchangeably used and defined in the broadest sense, and refers to moving any substance from inside a cell to outside a cell.

As used herein, a "conductive macromolecule" is defined in the broadest sense and refers to any macromolecule with conductivity. In this regard, conductivity refers to a property of conducting electricity that is known in the art. A macromolecule is also known as a polymer, referring to a molecule with a large molecular weight (generally defined as a molecular weight of 10000 or greater). Conductive macromolecules represented by polyacetylene change from insulator to semiconductor as of synthesis, and change from semiconductor to good conductor after an operation known as doping described below, such that electricity is conducted while being a macromolecule. The backbone of a conductive macromolecule such as polyacetylene generally has a conjugated structure with alternating repeats of a single bond and double bond ($\sigma$ bond + $\Pi$ bond). Such a conjugated structure is understood to affect whether a macromolecule is conductive. Impartation of conductively requires an operation (or reaction) known as doping. This is also referred to as chemical doping. The actual operation adds a small amount of a reagent (accepter that readily accepts an electron or a donor that readily provides an electron) to a conductive macromolecule to express conductivity. Doping can also be electrochemically performed. In such a case, it is understood that applying a voltage in an electrolytic solution with an electrolyte dissolved therein by using a conductive macromolecule as a positive or negative electrode results in accepter doping at the positive electrode and donor doping at the negative electrode.

Examples of conductive macromolecules include the following.

Conjugated system macromolecule Representative example

| Aliphatic conjugated system | polyacetylene |
| Aromatic conjugated system | poly(p-phenylene) |
| Mixed conjugated system | poly(p-phenylene vinylene) |
| Heterocyclic conjugated system | polypyrrole, polythiofene, polyethylene dioxythiophene (PEDOT) |
| Heteroatom-containing conjugated system | polyaniline |
| Polychain conjugated system | polyacene (virtual molecule) |
| Two dimensional conjugated system | graphene |

Preferred embodiments of conductive macromolecules include PEDOT. Research and development of PEDOT is ongoing worldwide in recent years. PEDOT is, in some aspects, one of the ideal forms of conductive macromolecules and is noted for its very high stability, high conductivity, pore injectability, and doping properties, but the present disclosure is not limited thereto. It is understood that conductive macromolecule thin membrane of any thickness can be readily made through spin coating with a solution prepared from dispersing PEDOT in water or an organic solvent with polystyrenesulfonate (PSS) as a macromolecule dopant (commonly known as PEDOT/PSS (e.g., available as Aldrich product number: 483095 or 560596)). It is understood in the art that a homogeneous membrane can be made with aligning the particle size of PEDOT. Thus, PEDOT can be advantageous in this regard.

In a preferred embodiment, a conductive macromolecule can have a property of expanding and contracting when a voltage is applied.

As used herein, "property of expanding and contracting when a voltage is applied", in the context of a conductive macromolecule, etc., refers to a property of a volume thereof expanding or contacting when a voltage is applied, and its reverse when application is discontinued (conductive macromolecule with an expanding volume when a voltage is applied contracts when application of voltage is discontinued, and conductive macromolecule with contracting volume expands when application is discontinued). Examples of conductive macromolecules having such a property include, but are not limited to, poly(3,4-ethylene dioxythiophene) (PEDOT), polythiophene, polyacetylene, polyaniline, polypyrrole, combinations thereof, etc.

As used herein, a "conductor" is defined in the broadest sense and refers to any object through which electricity passes. Representative examples include, but are not limited to, metals and semiconductors, preferably metals. A metal is preferred for the body of a tubular body. Examples of semiconductors include silicon. In a preferred embodiment, a conductive macromolecule does not constitute the body of a tubular body, but instead can be utilized as a coating.

Metals used herein are preferably metals that can be applied by electroless plating because it is advantageous that a conductive macromolecule can be plated without electroplating. Examples of metals that can be applied by electroless plating include, but are not limited to, gold, platinum, silver, and nickel, and alloys thereof.

As used herein, "electrode" refers to a pole for imparting electricity, and can be comprised of any conductive object. As long as a voltage can be applied to a conductive macromolecule and a storage unit, any shape can be used. Generally, two electrodes are provided, whereby electricity is applied. Meanwhile, one or three or more electrodes may be provided to the device of the present disclosure as needed.

As used herein, "substrate" consists of any shape and material, on which the tubular body of the present disclosure is disposed. In a preferred embodiment, a substrate can be configured into any shape after the tubular body of the present disclosure is disposed. A substrate preferably has a material and shape that would not pose a problem in accessing a cell. A substrate can be comprised of, for example, polycarbonate and/or polyethylene terephthalate, etc., but the material is not limited thereto.

As used herein, "storage unit" is a portion for storing a substance and is disposed to be communicable with a tubular body and a substrate. Preferably, the storage unit has a structure, which has an inside that is a void, is directly connected to the substrate, and has a tube through which an electrode can be inserted into the void. A storage unit is comprised of, for example, glass, ceramic, macromolecule, and/or metal, etc.

As used herein, "voltage supplying unit" can be any unit as long as a voltage can be supplied. Electricity may be supplied from a dry cell or rechargeable battery, or from an electric outlet of a house.

As used herein, "differential interference contrast prism" is also known as a Nomarski prism or a Nomarski-modified Wollaston prism, and refers to a prism that resolves a light into two polarized lights with oscillation surfaces that are orthogonal to each other. A Wollaston prism is prepared from two birefringent crystals such as calcite pasted together with offset crystalline axes. Such a prism can resolve light by the difference in indices of refraction based on the polarity of light. A Wollaston prism modified Nomarski prism has a focus of two resolved polarized lights outside of the prism. For this reason, an image formation position of a prism can be matched to an image formation surface of a condenser, which is understood to enable a dramatically more flexible optical path design. The optical path of a differential interference contrast microscope is configured by adding two polarizing plates and two Nomarski prisms to a normal bright field microscope. This can be used as a polarizing microscope by removing only the Nomarski prisms. Specifically, the present disclosure can further comprise an apparatus that can be utilized as a polarizing microscope.

(Preferred Embodiments)

Preferred embodiments of the present invention are described below. Embodiments described below are provided to facilitate the understanding of the present invention. It is understood that the scope of the present invention should not be limited to the following descriptions. Thus, it is apparent that those skilled in the art can make appropriate modifications within the scope of the present invention by referring to the descriptions herein. It is also understood that the following embodiments of the present invention can be used independently or as a combination thereof.

(1) Tubular body coated with a conductive macromolecule

The present disclosure provides a tubular body for introducing a substance into a cell and/or recovering a substance from a cell. The tubular body is coated with a conductive macromolecule. A tubular body coated with a conductive macromolecule can turn a flow of a substance passing through inside the tubular body on/off by applying a voltage. Since a flow of substance can be turned on/off, an undesirable outflow of intracellular substance from a cell can be stopped, so that the tubular body can be inserted for a long period of time.

In one aspect, the present disclosure provides a tubular body coated with a conductive macromolecule for introducing a substance into a cell and/or recovering a substance from a cell. In another aspect, use of a tubular body coated with a conductive macromolecule for introducing a substance into a cell and/or recovering a substance from a cell is provided.

One advantageous embodiment of the present disclosure has demonstrated that long-term introduction and recovery of a substance from applying a minute voltage ($\pm$ 0.1 V or less, a potential lower than the membrane potential) can be materialized by inserting a metal nanotube coated with a conductive macromolecule into a cell (Figure **13**). In an exemplary embodiment, a tubular body that is a metal nanotube shown herein is comprised of gold, platinum, etc. exhibiting an outer diameter of, for example, 0.3 to 2.0 $\mu$m, an inner diameter of, for example, 0.1 to 1.9 $\mu$m, and a height (length) of 1 to 40 $\mu$m. Conventional technologies materialize introduction and recovery of a substance from puncturing a hole in a cell membrane by applying a high voltage (about 0.1 to 10 V) by a method so-called electroporation without inserting a nanotube into a cell. However, application of a high voltage to cells results in an undesirable change in cellular function such as cell death or oncogenesis. In this regard, the present disclosure developed a metal nanotube coated with a conductive macromolecule to materialize insertion of a nanotube into a cell for an extended period, and controls transport and recovery of a substance by applying a minute voltage to the nanotube. This could not have been achieved with conventional technologies.

In the present disclosure, a tubular body is generally made of a conductor. Since a tubular body is coated with a conductive macromolecule, the conductor used at the body portion is preferably a material that is not a conductive macromolecule such as metal. Conductors are preferably metals, including materials that can be applied by electroless plating such as gold, platinum, silver, and/or nickel.

In one preferred embodiment, the conductive macromolecule has a property of expanding and contracting when a voltage is applied. Examples of preferred conductive macromolecules include, but are not limited to, poly(3,4-ethylene dioxythiophene) (PEDOT), polythiophene, polyacetylene, polyaniline, polypyrrole, and combinations thereof.

Coating the tubular body of the present disclosure with a material that can expand and contract from applying a voltage has enabled controlling movement, i.e., introduction and recovery, of a substance into/from a cell by applying a voltage or discontinuing the application. This could not have been achieved with conventional technologies.

In one preferred embodiment, the voltage is 500 mV or less, 400 mV or less, 300 mV or less, 200 mV or less, 100 mV or less, 90 mV or less, 80 mV or less, 70 mV or less, 60 mV or less, 50 mV or less, 40 mV or less, 30 mV or less, 20 mV or less, or 10 mV or less. If the voltage is about 100 mV or less, the viability rate of cells is relatively high, and if the voltage is 70 mV or less, the viability rate is nearly 100%. Meanwhile, since movement of a substance in and out can be achieved with a nearly 100% efficiency, one of the significance of the present disclosure is in materializing movement of a substance into/from a cell even with such a minute voltage.

In the present disclosure, a voltage may be AC or DC. Preferably, an AC voltage can be used. Although not wishing to be bound by any theory, this is because the use of alternating current increases the efficiency of moving a substance in and out and/or increases the viability rate, but the voltage is not limited thereto.

In a preferred embodiment, the tubular body has an inner diameter of 3 $\mu$m or less, 2 $\mu$m or less, or 1 $\mu$m or less. Such an inner diameter may be changed depending on the substance to be moved in and out of interest. If an organelle such as mitochondria is to be moved in and out, a tubular body can have an inner diameter of 2 $\mu$m or less.

In a preferred embodiment, the tubular body has an outer diameter of 5 $\mu$m or less, 4 $\mu$m or less, or 3 $\mu$m or less. Such an outer diameter may be changed depending on the cell of interest to/from which a subject is to be moved. If an organelle such as mitochondria is to be moved in and out, a tubular body can have an outer diameter of 4 $\mu$m or less.

In one preferred embodiment, the tubular body has a length of 5 $\mu$m or greater and 50 $\mu$m or less. For example, the length can be 6 $\mu$m or greater, 7 $\mu$m or greater, 8 $\mu$m or greater, 9 $\mu$m or greater, 10 $\mu$m or greater, 15 $\mu$m or greater, 20 $\mu$m or greater, 25 $\mu$m or greater, 30 $\mu$m or greater, 35 $\mu$m or greater, 40 $\mu$m or greater, or 45 $\mu$m or greater, and 49 $\mu$m or less, 48 $\mu$m or less, 47 $\mu$m or less, 46 $\mu$m or less, 45 $\mu$m or less, 40 $\mu$m or less, 45 $\mu$m or less, 40 $\mu$m or less, 35 $\mu$m or less, 30 $\mu$m or less, 25 $\mu$m or less, 20 $\mu$m or less, 15 $\mu$m or less, or 10 $\mu$m or less. The length can be within any range between these values. The length may be changed depending on the cell of interest to/from which a subject is to be moved. Although not wishing to be bound by any theory, this is because such lengths are advantageous for the configuration into a stamping system.

In a preferred embodiment, the substance has an arithmetic mean diameter (typically a volume mean diameter) of 1 nm or greater and 2 $\mu$m or less. For example, the arithmetic mean diameter can be 5 nm or greater, 10 nm or greater, 15 nm or greater, 20 nm or greater, 25 nm or greater, 30 nm or greater, 35 nm or greater, 40 nm or greater, 45 nm or greater, 50 nm or greater, 60 nm or greater, 70 nm or greater, 80 nm or greater, 90 nm or greater, 100 nm or greater, 150 nm or greater, 200 nm or greater, 250 nm or greater, 300 nm or greater, 350 nm or greater, 400 nm or greater, 450 nm or greater, 500 nm or greater, 600 nm or greater, 700 nm or greater, 800 nm or greater, 900 nm or greater, 1000 nm or greater, 1100 nm or greater, 1200 nm or greater, 1300 nm or greater, 1400 nm or greater, 1500 nm or greater, 1600 nm or greater, 1700 nm or greater, 1800 nm or greater, or 1900 nm or greater, and 2 $\mu$m or less, 1900 nm or less, 1800 nm or less, 1700 nm or less, 1600 nm or less, 1500 nm or less, 1400 nm or less, 1300 nm or less, 1200 nm or less, 1100 nm or less, 1000 nm or less, 900 nm or less, 800 nm or less, 700 nm or less, 600 nm or less, 500 nm or less, 400 nm or less, 300 nm or less, 200 nm or less, 100 nm or less, 90 nm or less, 80 nm or less, 70 nm or less, 60 nm or less, 50 nm or less, 40 nm or less, 30 nm or less, 20 nm or less, 10 nm or less, or 5 nm or less. The arithmetic mean diameter can be within any range between these values. Such suitable diameters may be changed depending on the cell of interest to/from which a subject is to be moved. The present disclosure is advantageous in that a relative large size corresponding to a nucleic acid, peptide, protein, or organelle can be used.

In a preferred embodiment, the substance comprises at least one selected from the group consisting of a low molecular weight compound, a nucleic acid, a peptide, a protein, and an organelle.

The present disclosure can extend the period during which a nanotube is inserted into a cell. Furthermore, a change in the volume of a conductive macromolecule can be induced by applying a lower voltage than a membrane potential to control transport of a substance into a cell as desired.

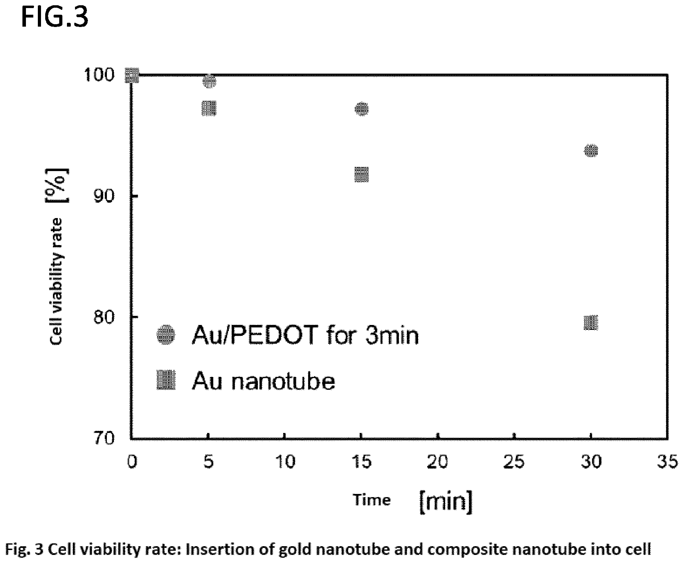

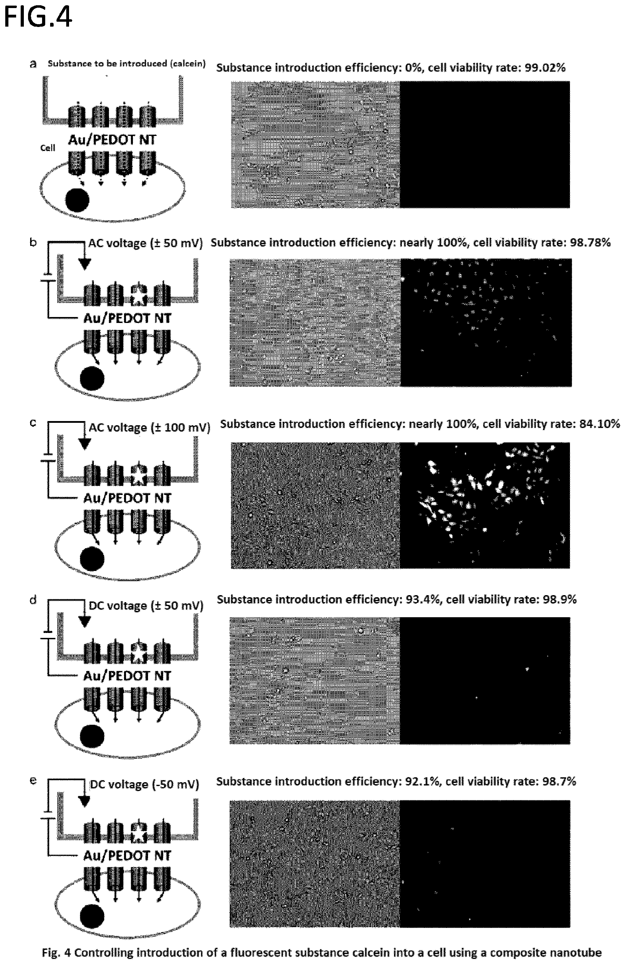

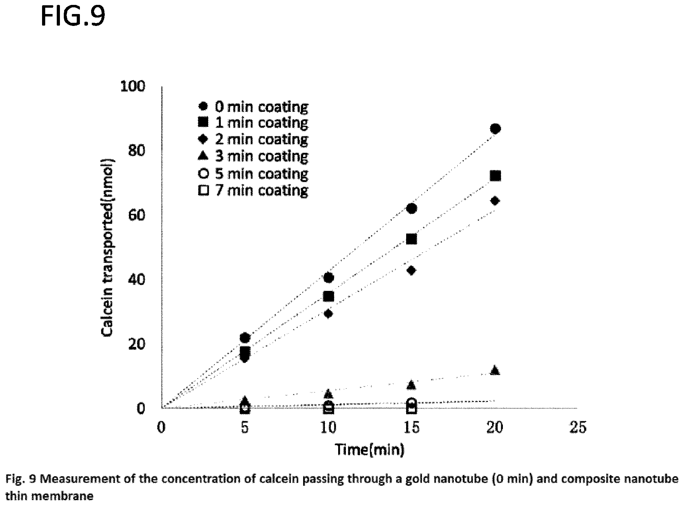

Although not wishing to be bound by any theory, if only a metal nanotube is inserted into a cell, intracellular substances are generally released outside, so that the cell dies over time (Figure **3**). If a metal nanotube is coated with a conductive macromolecule membrane, release of substance is suppressed to improve the viability rate of cells. Furthermore, release of substances from a composite nanotube can be controlled by turning electricity On/Off (see the Examples and Figure **9**). Transport of substance could not be controlled by turning electricity On/Off with only a conventionally used gold nanotube. As a result of evaluation using low molecular weight calcein (622.55 g/mol in the Examples) as a model substance and a human derived cancer cell (HeLa), transport of the substance into a cell could not be observed when electricity was Off, but a calcein stained cell was found when, for example, ± 50 mV was applied as shown in the Examples (see the Examples and Figure **4**). After applying electricity, the viability rate of cells exhibited a high value of 98.78%. It was found that the present invention can control intracellular transport by applying electricity, even if the substance to be introduced is changed to a macromolecule (green fluorescent protein, 26.9 kDa) and exhibits a high viability rate. Interestingly, it was found that the viability rate decreases if the applied voltage is a voltage of the cell membrane potential (approximately 70 mV) or greater ± 100 mV as shown in the Examples (about 84.10% as shown in the Examples).

(2) Device

The present disclosure provides a device for introducing a substance into a cell and/or recovering a substance from a cell. The device comprises a tubular body and a substrate, and the tubular body is disposed on the substrate. A tubular body is coated with a conductive macromolecule. Introduction of a substance into a cell and/or recovery of a substance from a cell is facilitated by providing the present disclosure as a device.

In one aspect, the present disclosure provides a device for introducing a substance into a cell and/or recovering a substance from a cell, comprising a tubular body coated with a conductive macromolecule, a substrate, and an electrode, wherein the tubular body is disposed on the substrate.

The tubular body of the present disclosure penetrates through a substrate. A substance can be passed through the tubular body downward from the top surface of the substrate, and a substance can be suctioned upward through the tubular body. For individual tubular bodies, any one of the embodiments described in "(1) Tubular body coated with a conductive macromolecule" or a combination thereof can be used.

In a preferred embodiment, the device comprises a plurality of the tubular bodies.

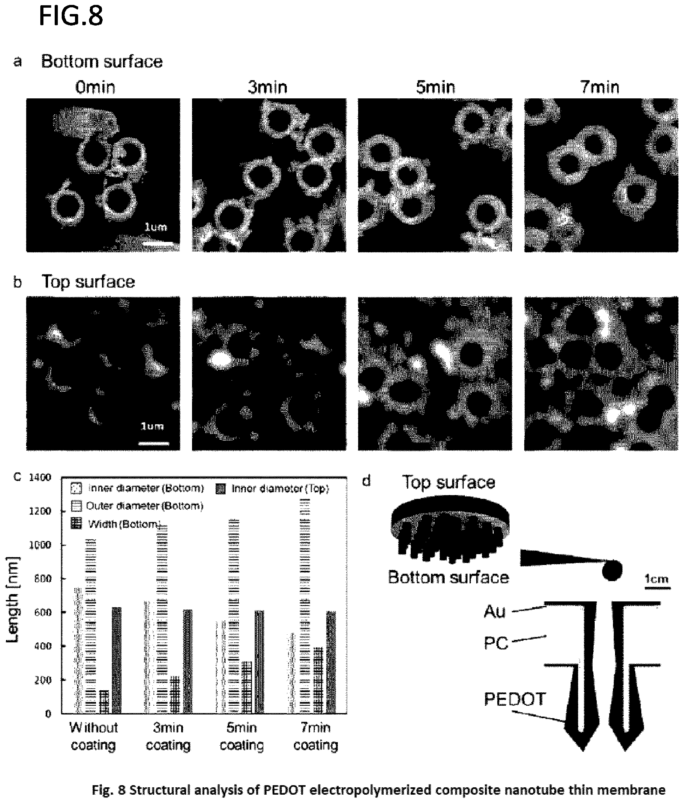

The tubular body of the present disclosure is disposed on the substrate of the present disclosure. Although not wishing to be bound by any theory, exemplary shapes of a substrate include membranous shapes as shown in Figure **8d.** A plurality of tubular bodies are disposed on the substrate, like a pin holder. While a tubular body is desirably disposed approximately vertically with respect to a substrate, the configuration is not limited thereto. An electrode can be disposed on a substrate.

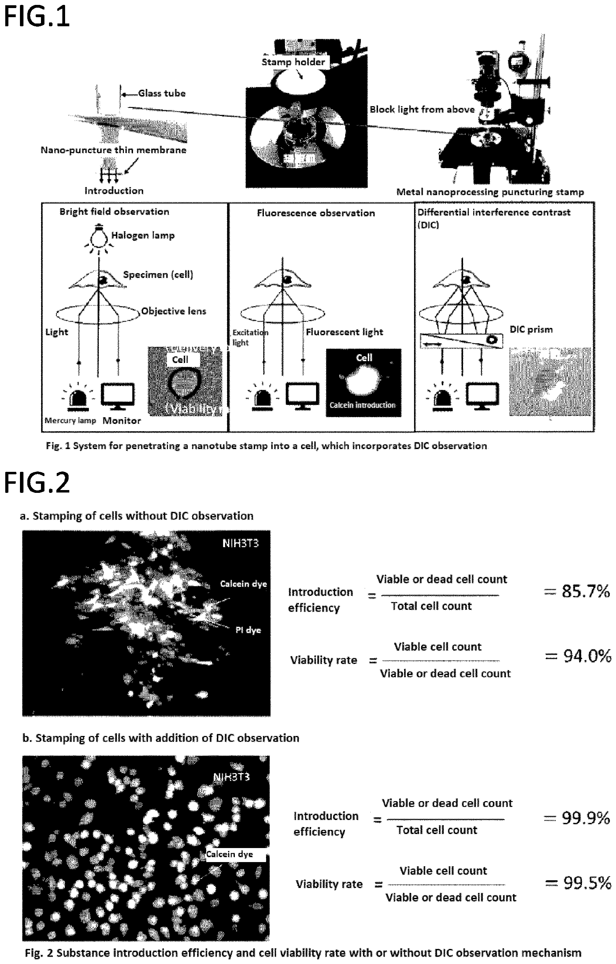

Therefore, an example of the device of the present disclosure includes those with a stamp shape (also referred to as a stamping system) as shown in Figure **15**. In Figure **15**, **1** indicates the handle of a stamp. **2** indicates a (substance) storage unit. **3** indicates a (membranous) substrate. **4** indicates a substance. **5** indicates a penetrating tubular body. A tubular body can be inserted or withdraw into/from a cell by manually holding or mechanically grasping the handle of a stamp.

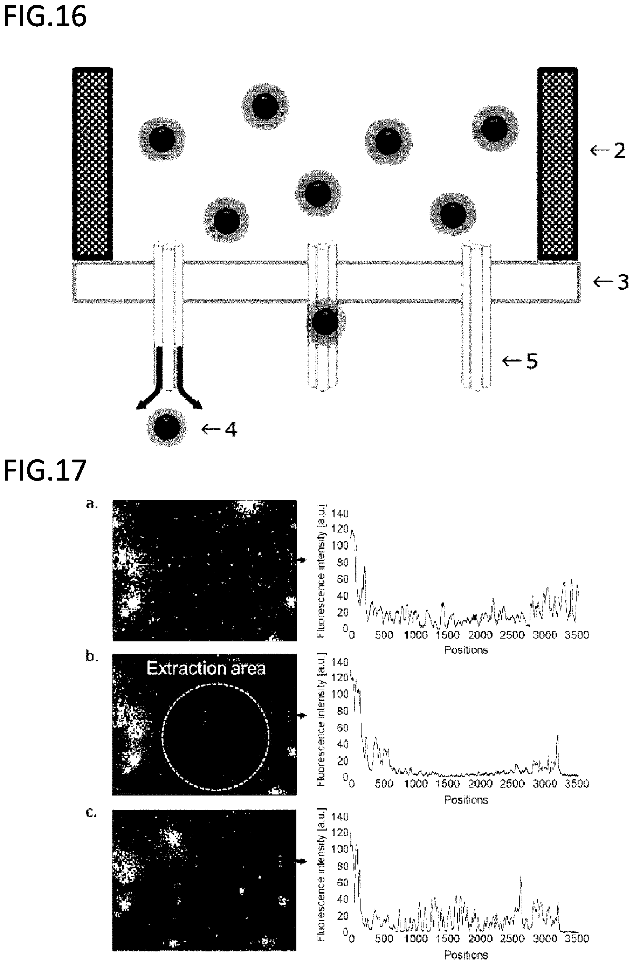

Figure **16** shows an expanded view of the storage unit **2**, substrate **3**, and tubular body **5** in Figure **15**. The substance **4** contained in the storage unit can be introduced into a cell through the tubular body **5** that penetrates through the substrate **3**. A substance in a cell can be recovered from inside the cell through the tubular body **5**. The storage unit **2** can contain the substance **4** and a solvent for dissolving or suspending the substance (e.g., water).

In a preferred embodiment, the tubular body and the substrate further comprise a storage unit for storing the substance. The storage unit may contain a substance to be introduced (e.g., low molecular weight compound, peptide, protein, nucleic acid, or organelle (mitochondria, etc.)). Besides the substances to be introduced, substances with the same component composition as the cell of interest may be filled. On the other hand, if recovery is intended, substances with components other than the substance to be recovered having the same component composition as the cell of interest can be stored.

In a preferred embodiment, the substrate is comprised of, but is not limited to, polycarbonate or polyethylene terephthalate.

In a preferred embodiment, the storage unit preferably has a structure, which has an inside that is a void, is directly connected to the substrate, and has a tube through which an electrode can be inserted into the void.

In a preferred embodiment, the storage unit is comprised of, but is not limited to, glass, ceramic, macromolecule, metal, etc. A macromolecule or other materials can be any material that can retain a solution within a storage unit. Examples of macromolecules include, but are not limited to, ABS resin, polyethylene (including those that are cross-linked), ethylene-vinyl acetate copolymer, acrylic resin, polyacrylic acid, polyamide (nylon, aramid, etc.), polybutylene terephthalate, polycarbonate, polyether ether ketone, polyester (polytrimethylene terephthalate, polyethylene naphthalate, polybutylene naphthalate, etc.), polyethylene, polyethylene terephthalate, polyimide, polylactic acid, polyacetal, polyphenylene ether, polypropylene, polystyrene, polyethersulfone, polytetrafluoroethylene, polyurethane, polyvinyl chloride, polyvinylidene chloride, styrene maleic anhydride, AS resin, etc. The storage unit may be fabricated from thick waterproof paper (cellulose).

(3) System

The present disclosure provides a system for introducing a substance into a cell and/or recovering a substance from a cell by applying a voltage to the device of the present disclosure.

In one aspect, the present disclosure provides a system for introducing a substance into a cell and/or recovering a substance from a cell, comprising the device of the present disclosure and a voltage supplying unit for supplying a voltage. It is understood that the device can have any shape described herein in "(1) Tubular body coated with a conductive macromolecule" or "(2) Device".

In a preferred embodiment, the system of the present disclosure further comprises a container comprising a cell. Any container can be used, as long as a cell can be maintained. A container may be made of sterilized plastic or glass material.

In one preferred embodiment, the system further comprises an observation unit for differential interference contrast observation of the cell in a container from below. For differential interference contrast observation, a microscope that is commonly used (e.g., polarizing microscope, etc.) can comprise, for example, a differential interference contrast prism (e.g., a prism also known as a Nomarski prism or a Nomarski-modified Wollaston prism, which resolves light into two polarized lights with oscillation surfaces that are orthogonal to each other). Such a prism is prepared from two birefringent crystals pasted together with offset crystalline axes. Light can be resolved by the difference in indices of refraction based on the polarity of light. The optical path of a differential interference contrast microscope is generally configured by adding two polarizing plates and two differential interference contrast prisms to a normal bright field microscope, and such a configuration can be used (e.g., optional mirror unit known as Olympus U-MDIC3 can be used).

In a preferred embodiment, the system further comprises a display unit for displaying an image from the observation unit. Any display, etc. can be used as such a display unit, as long as a microscope image can be displayed.

In a preferred embodiment, insertion of a tubular body contained in the device into the cell can be adjusted in units of 0.01 $\mu$m, 0.02 $\mu$m, 0.03 $\mu$m, 0.04 $\mu$m, 0.05 $\mu$m, 0.06 $\mu$m, 0.07 $\mu$m, 0.08 $\mu$m, 0.09 $\mu$m, 0.1 $\mu$m, 0.011 $\mu$m, 0.012 $\mu$m, 0.013 $\mu$m, 0.014 $\mu$m, 0.015 $\mu$m, 0.016 $\mu$m, 0.017 $\mu$m, 0.018 $\mu$m, 0.019 $\mu$m, 0.2 $\mu$m, 0.3 $\mu$m, 0.4 $\mu$m, 0.5 $\mu$m, 0.6 $\mu$m, 0.7 $\mu$m, 0.8 $\mu$m, 0.9 $\mu$m, or 1.0 $\mu$m.

(4) Introduction/recovery method

The present disclosure provides a method for introducing a substance into a cell and/or recovering (or retrieving) a substance from a cell. This can be performed using the device or system of the present disclosure. Introducing a substance into a cell and/or recovering a substance from a cell can be controlled by applying a voltage over a desired period of time.

In one aspect, the present disclosure provides a method for introducing a substance into a cell and/or recovering a substance from a cell, comprising the steps of: (a) providing the device or system of the present disclosure; and (b) applying a voltage to the tubular body over a desired period of time. Although not wishing to be bound by any theory, it is understood that a gate opens and a substance disperses and moves only when a voltage is applied. This enables introduction of a substance into a cell or recovery (retrieval) of a substance. For the device, system, and tubular body used in the present disclosure, it is understood that any embodiment described in "(1) Tubular body coated with a conductive macromolecule", "(2) Device", or "(3) System" or a combination thereof can be used.

In the present disclosure, the period during which a voltage is applied to a tubular body can be appropriately adjusted depending on the desired degree of achievement of introduction of a substance and/or recovery of a substance. The period is also dependent on voltage. The period can also be dependent on the type of voltage (AC voltage or DC voltage, etc.)

The following may also need to be heeded in the introduction/recovery method of the present disclosure. For the viability rate, sterilization and deaeration of bubbles within a needle prior to penetration, rate of penetration into a cell and depth reached by penetration, suppression of vibrations during a substance introduction process, etc. should be noted, but factors to be noted are not limited thereto. For the recovery rate, sterilization and deaeration of bubbles within a needle prior to penetration, rate of penetration into a cell and depth reached by penetration, suppression of vibrations during a substance introduction process, etc. should be noted, but factors to be noted are not limited thereto. For the size of a substance of interest, small molecules such as fluorescent dyes have a risk of discoloration or deterioration due to exposure to light, so that storage in a dark room and manipulation at a suitable temperature, etc. are desirable, but factors to be noted are not limited thereto. Furthermore, proteins, organelles, etc. are delicate materials that are readily inactivated from vibration or process of manipulation. Although not wishing to be bound by any theory, this is because a large molecular size slows down dispersion of a substance, so that introduction and recovery time tends to be long.

(5) Device manufacturing method

The present disclosure provides a method of fabricating a device comprising a tubular body coated with a conductive macromolecule and a substrate.

In one aspect, the present disclosure provides a method of fabricating a device comprising a tubular body coated with a conductive macromolecule and a substrate, comprising the step of: securing a membrane of the conductive macromolecule to a substrate on which the tubular body is disposed at preferably two or more points to coat the tubular body.

In a preferred embodiment, the step of coating can be achieved by providing working electrodes that contact the substrate at two or more points. Although not wishing to be bound by any theory, this is because it was found that stable On/Off is achieved by providing securing portions of working electrodes at two or more points. It is understood that the present disclosure is not limited thereto.

In a preferred embodiment, the substrate on which the tubular body is disposed is fabricated by a process comprising the step of electroless plating of a metal on track-etched polycarbonate and the step of etching a metal plated surface.

In a preferred embodiment, an inner diameter of a metal straw coated with the conductive macromolecule is 0.01 $\mu$m to 2 $\mu$m, and an outer diameter is 0.1 $\mu$m to 5 $\mu$m.

(Method of activating a cell)

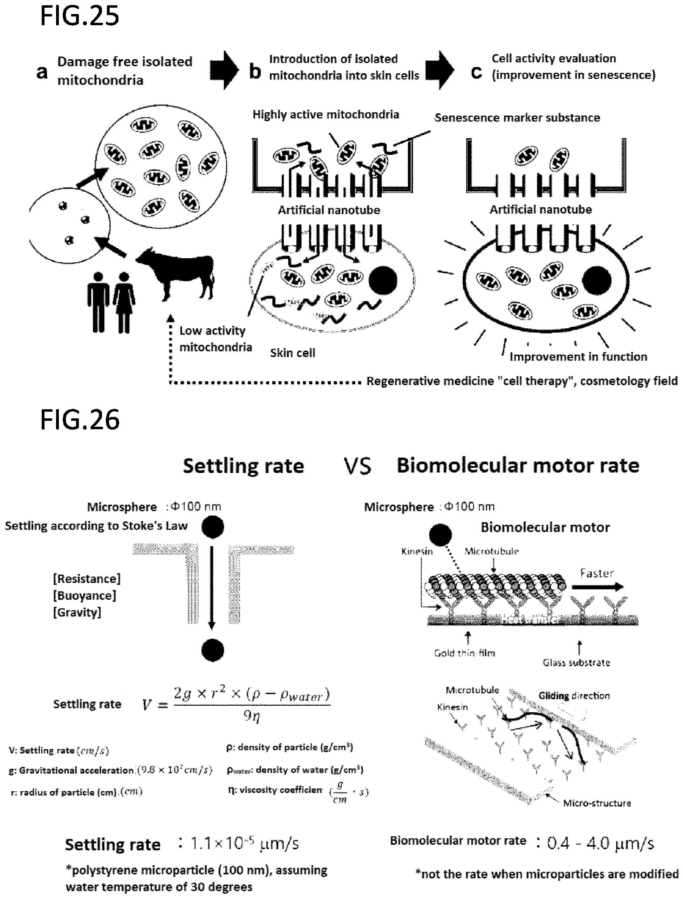

The present disclosure provides a method of activating a cell. The method comprises the step of introducing a mitochondrion into the cell by using the device or system of the present disclosure. The device and system of the present disclosure can introduce a macromolecule exceeding 1 MDa (such as a mitochondrion) efficiently and safely into a cell. An antiaging effect can be expected by introducing a fresh mitochondrion into a cell. The method, device, and system of the present disclosure are advantageous in terms of being simple and less likely to result in cell death when introducing a mitochondrion into a cell. Since a fresh mitochondrion can be introduced into an organ in organ transplant or a cell sheet, cell activation that is beneficial especially in the fields of regenerative medicine (cell therapy), cosmetology, etc. can be achieved (Figure **25**). Meanwhile, cells that continue to grow such as cancer cells have a reduced mitochondrial function, so that cancer cells can be killed by introducing fresh mitochondria.

(Method of controlling cellular function)

The present disclosure provides a method of controlling a cellular function. The method comprises the step of introducing a gene product (protein) into the cell by using the device or system of the present disclosure. The method can directly induce (direct reprogramming) a cellular function by introducing a gene product into a cell. Somatic cells can be directly induced into specific differentiated cells.

In a preferred embodiment, the gene product comprises gene products from inducing differentiation of cells, e.g., reprogramming factors such as Sox2, Klf4, c-Myc, and Oct4. Factors such as Oct4 are one type of transcription factor. It is known that induction of differentiation of cells can be controlled by controlling the expression level thereof. Induction of differentiation of cells can be controlled by the method, device, or system of the present disclosure.

(Intracellular transport mechanism using a biomolecular motor)

The present disclosure provides an intracellular transport mechanism using a biomolecular motor. When a microparticle, etc. is introduced into a cell by settling, the settling rate would be about $1.1 \times 10^{-5}$ $\mu$m/s. Meanwhile, a molecular motor rate of 0.4 to 4.0 $\mu$m is expected with a biomolecular motor using a combination of kinesin and a microtubule (Figure **26**). A substance can be introduced into a cell quickly and safely by the biomolecular motor of the present disclosure. Besides kinesin molecules, dynein can also be used. Furthermore, a transport function using an actin filament and myosin can be comprised as a mechanism for accelerating transport.

The present disclosure provides a tubular body coated with a conductive macromolecule for introducing a substance into a cell and/or recovering a substance from a cell, wherein the conductive macromolecule comprises kinesin. Such a tubular body can be used as a biomolecular motor in combination with a microtubule.

The present disclosure provides use of a tubular body coated with a conductive macromolecule comprising kinesin for introducing a substance into a cell and/or recovering a substance from a cell.

The present disclosure provides a device for introducing a substance into a cell and/or recovering a substance from a cell, comprising a tubular body coated with a conductive macromolecule comprising kinesin, a substrate, and an electrode, wherein the tubular body is disposed on the substrate.

The present disclosure provides a system for introducing a substance into a cell and/or recovering a substance from a cell, comprising: the device of the present disclosure; and a voltage supplying unit for supplying a voltage.

The present disclosure provides a method for introducing a substance into a cell and/or recovering a substance from a cell, comprising the steps of:

(a) providing the device or system of the present disclosure;
(b) providing a microtube with a substance attached thereto; and
(c) applying a voltage to the tubular body over a desired period of time.

A substance that can be introduced in this regard can be any substance. Substances having especially the following properties can be suitably introduced/recovered. While almost all intracellular content except for the nucleus can be recovered, it is understood that smaller substances can be recovered at a greater quantity from the viewpoint of substance transport rate. Specifically, sugars, lipids, proteins (amino acids), and nucleic acids can be primarily introduced/recovered. Besides small molecules, organelles (vesicle, Golgi apparatus, mitochondria, etc.) can also be directly introduced/recovered.

Tubular bodies that are used are described herein, but suitable examples are the following. For introduction/recovery of small molecules, a diameter of about 50 to 400 nm is preferred. For middle molecules such as proteins, a diameter of about 400 to 1000 nm is preferred. For larger organelles, a diameter of about 1000 to 1500 nm is preferred.

The present disclosure provides a method of fabricating a tubular body coated with a conductive macromolecule comprising kinesin, comprising the step of attaching kinesin to the tubular body.

(Stamping system)

The present disclosure provides a stamping system for inserting a composite nanotube into a cell, comprising:

a stamp comprising a tubular body coated with a conductive macromolecule;
an electrode; and
a voltage supplying unit for supplying a voltage. This system can be equipped with a microscope and can readily and safely introduce a substance into a cell by using the microscope.

Examples of substances that can be introduced/recovered in a stamping system include intracellular content excluding the nucleus. Almost all content can be recovered. It is understood that smaller substances can be recovered at a greater quantity from the viewpoint of substance transport rate. Sugars, lipids, proteins (amino acids), and nucleic acids can be primarily introduced/recovered. Besides small molecules, organelles (vesicle, Golgi apparatus, mitochondria, etc.) can also be directly introduced/recovered.

Optimal examples of a voltage supplying unit include bipolar power sources that can apply a voltage of about the cell membrane potential and tripolar electrochemical power sources that can accurately control the potential in a solution.

The present disclosure provides a penetration observation system for inserting a composite nanotube into a cell, comprising:

the device of the present disclosure;
a voltage supplying unit for supplying a voltage; and
a microscope.

Examples of substances that can be introduced/recovered in an observation system include intracellular content excluding the nucleus. Almost all content can be recovered. It is understood that smaller substances can be recovered at a greater quantity from the viewpoint of substance transport rate. Sugars, lipids, proteins (amino acids), and nucleic acids can be primarily introduced/recovered. Besides small molecules, organelles (vesicle, Golgi apparatus, mitochondria, etc.) can also be directly introduced/recovered. Optimal examples of a voltage supplying unit include bipolar power sources that can apply a voltage of about the cell membrane potential and tripolar electrochemical power sources that can accurately control the potential in a solution.

The present disclosure provides a composite nanotube thin membrane and stamp kit for delivering a substance into a cell or extracting a substance from a cell, comprising:

a composite nanotube thin membrane comprising a tubular body coated with a conductive macromolecule; and
a stamp.

A substance can be introduced into a cell to control a function of the cell or to induce the cell to manufacture a substance. By extracting an intracellular substance, the extracted substance can be utilized in functional analysis of the cell.

In this manner, the present disclosure developed a metal nanotube coated with a conductive macromolecule (composite nanotube) to materialize insertion of a nanotube into a cell for an extended period, and highly efficient transport and extraction of a substance by applying a minute voltage to the nanotube by using the kit of the present disclosure. Furthermore, a (microscope equipped) stamping system for inserting such a composite nanotube into a cell can be provided. For example, an information transmitting substance can be safely and efficiently provided.

The present disclosure is expected to be utilized in the field of life science, and is applicable in technologies for measuring or controlling cell activity. Introduction of a substance into a cell and extraction of intracellular information substance can be materialized by using the technology of the present disclosure. The technology of the present disclosure is applicable in measurement/control technologies utilized in basic science or medical field, or regenerative medicine for creating an organ from cells, or engineering field using cells such as initiatives for cultured food for creating meat from cells.

(Method of evaluating cell activity)

The present disclosure provides a method of evaluating cell activity, comprising the step of:
measuring a quantity of a marker substance within a cell by using a tubular body coated with a conductive macromolecule. Examples of the marker substance include, but are not limited to, gene products (proteins). Examples include substances that are well known as an indicator of cell activity.

In a preferred embodiment, the method further comprises the step of introducing a mitochondrion into a cell.

(Method of introducing a cell using a microsphere)

The present disclosure provides a method of introducing a cell using a microsphere, comprising the steps of:

(a) providing the device or system of the present disclosure;
(b) providing a microsphere; and
(c) applying a voltage to the tubular body over a desired period of time.

In a preferred embodiment, the method further comprises the step of attaching a substance to the microsphere.

(Method of extracting an intracellular substance)

The present disclosure provides a method of extracting an intracellular substance, comprising the steps of:

(a) providing the device or system of the present disclosure; and
(b) applying a voltage to the tubular body over a desired period of time.

Examples of the intracellular substance include, but are not limited to, proteins, nucleic acids, lipids, saccharides, complexes thereof, organelles, etc.

(Cell reprogramming method)

The present disclosure provides a cell reprogramming method, comprising the steps of:

(a) providing the device or system of the present disclosure;
(b) providing a gene or a gene product; and
(c) applying a voltage to the tubular body over a desired period of time.

Examples of the gene include DNA, RNA, derivatives thereof, etc. Examples of the gene product include, but are not limited to, mRNA, proteins, proteins modified post-translation, etc. Metabolites can also be used.

In this manner, the present disclosure can freely design and fabricate a cell by programming the cell, and materialize a high level biological cell (smart cell). Such a cell can be used in manufacturing, food industry, environment, medicine, etc. by utilizing cell reprogramming. Such a cell can manufacture cultured food products, pharmaceutical products, or macromolecules or manufacture cultured tissue, and is useful in the field of regenerative medicine or cosmetology. A plurality of substances can be safely introduced/extracted a plurality times into/from many cells to enable function control, substance manufacture, function analysis, or substance use. The present disclosure enables in cell NMR in a cellular environment. Cell senescence can be improved, and cells that have become cancerous can be killed. Mitochondria can also be introduced. The present disclosure enables controlling the cellular function and direct induction of differentiated cells (direct reprogramming). The present disclosure is useful in pharmaceuticals, industrial products, seasoning, fragrance, etc. as smart cells.

(Method of modifying the phenotype of a cell)

The present disclosure provides a method of introducing a functional protein into a cell to modify a phenotype of the cell, comprising the steps of:

(a) providing the device or system of the present disclosure;

(b) providing a functional protein; and

(c) applying a voltage to the tubular body over a desired period of time.

Examples of the functional protein include, but are not limited to, various enzymes including proteins, e.g., reprogramming factors such as Sox2, Klf4, c-Myc, and Oct4, antibodies such as anti-Bcr-Abl, enzymes and enzyme groups associated with metabolism.

In this manner, the present disclosure is useful in providing a high level biological cell (smart cell). Such a cell can be used in manufacturing, food industry, environment, medicine, etc. by utilizing modification of the phenotype of a cell. Such a cell can manufacture cultured food products, pharmaceutical products, or macromolecules or manufacture cultured tissue, and is useful in the field of regenerative medicine or cosmetology. A plurality of substances can be safely introduced/extracted a plurality times into/from many cells to enable function control, substance manufacture, function analysis, or substance use. The present disclosure enables in cell NMR in a cellular environment. Cell senescence can be improved, and cells that have become cancerous can be killed. Mitochondria can also be introduced. The present disclosuree enables controlling the cellular function and direct induction of differentiated cells (direct reprogramming). The present disclosure is useful in pharmaceuticals, industrial products, seasoning, fragrance, etc. as smart cells.

(Fabrication of a substrate comprising a tubular body)

Production of a substrate is described hereinafter as an exemplary embodiment.

A substrate (e.g., track-etched polycarbonate (TEPC) template) is subjected to electroless plating with a metal (e.g., Au), and then the top surface is etched (Figure **11a**). Electroless plating of a metal (e.g., Au) nanolayer is applied on a porous membrane. This process is a sufficiently established plating process consisting of four steps: (1) sensitization, (2) activation, (3) displacement plating, and (4) electroless plating (Figure **12**). In order to activate the surface of a substrate (e.g., TEPC membrane (e.g., pore size: 600 nm, thickness: 23 $\mu$m)), the substrate (e.g., TEPC membrane) is immersed in, for example, a 10.55 mM $SnCl_2$ solution at 24°C, and then immersed in a 11.28 mM $PdCl_2$ solution at 24°C. After washing the membrane with water, a tin-palladium metal layer is formed on the TEPC surface and used as a catalyst for electroless plating. The amount of metal catalyst on the TEPC surface is controlled by adjusting the immersion time and number of activation cycles. Subsequently, the substrate coated with a catalyst (e.g., TEPC membrane) is immersed in a 2 g/L metal (e.g., gold) plating solution for 24 hours at 40°C. After coating the membrane with a metal (e.g., Au) nanolayer, the color of the substrate (e.g., TEPC membrane) (Figure **11a**) changes to the color of the metal (e.g., gold) on both the top surface and the bottom surface (Figure **11b**). It can be confirmed that a nanotube with a metal (e.g., Au) on the inside is formed from investigating the metal/substrate (e.g. Au/TEPC membrane) by using a scanning electron microscope (SEM).

After the metal/substrate (e.g., Au/TEPC membrane) is formed, only the top surface of the metal (e.g., Au) plated nanolayer is etched with aqua regia (mixture of nitric acid and hydrochloric acid at a molar ratio of 1:3), and both the substrate material (e.g., polycarbonate) and metal (e.g., Au) nanotube inside are exposed on the membrane surface (Figures **11a** and **11e**). After wet etching, a change in color from a metal color (e.g., gold) to for example brown can be observed only on the etched surface (Figure **11d**). The outer diameter and inner diameter of the exposed metal (e.g., Au) nanotube can be checked by using an SEM (Figure **11e**). When immersed in a metal (e.g., gold) plating solution for 24 hours, a 100 $\pm$ 30 nm metal (e.g., Au) layer can be formed on the substrate (e.g., TEPC). If the $O_2$ plasma-exposed substrate (e.g., TEPC) is further etched, a hollow metal (e.g., Au) nanoneedle (nanostraw) is generated. The height (H) of a metal (e.g., Au) nanostraw can be controlled by adjusting $O_2$ plasma exposure time: e.g., 0 min (Figure **11e**, H: 0 $\mu$m), 10 min (Figure **11f**, H: 1.3 $\mu$m), 20 min (Figure 2g, H: 2.4 pm), or 30 min (Figure 2h, H: 5.0 $\mu$m).

(Method of electropolymerization of conductive macromolecule monomer on a tubular body)

A tubular body can be coated with a conductive macromolecule by electropolymerization of the conductive macromolecule on a substrate comprising a tubular body fabricated as described above. Electropolymerization may be performed under conditions that are generally used for the conductive macromolecule. For example for PEDOT, a conductive macromolecule is deposited on a metal surface by applying a constant voltage of 1 V for a certain period of time in a polymerization solvent (aqueous mixture comprising a 50 mM EDOT ($C_6H_6O_2S$) monomer and dopant (100 mM $KNO_3$ or 100 mM $LiCiO_4$)). The application period depends on the area or shape of exposed gold, but is about 1 to 10 minutes.

(Application Example)

The present disclosure enables introduction of any substance safely into a cell. The present disclosure can be used in regenerative medicine/therapy for the creation of an organ/tissue from a cell unit and can be applied to food industry such as cultured cell meat or plant.

(Note)

As used herein, "or" is used when "at least one or more" of the listed matters in the sentence can be employed. When explicitly described herein as "within the range" of "two values", the range also includes the two values themselves.
Reference literatures such as scientific literatures, patents, and patent applications cited herein are incorporated herein by reference to the same extent that the entirety of each document is specifically described.
As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. While the present disclosure is described more specifically with Reference Examples, Examples, and Test Examples hereinafter, the descriptions are provided for the sole purpose of exemplification and the present disclosure is not limited thereto. The compound names denoted in the following Reference Examples and Examples do not necessarily follow the IUPAC nomenclature. While abbreviations are sometimes used to simplify a description, these abbreviations are defined the same as the above descriptions. The scope of the present disclosure is not limited to the embodiments and Examples specifically described herein and is limited only by the scope of claims.

[Examples]

Examples are described hereinafter. The cells used in the following Examples were handled in compliance with the law and regulation of Waseda University and others. For reagents, the specific products described in the Examples were used. However, the reagents can be substituted with an equivalent product from another manufacturer (Sigma-Aldrich, Wako Pure Chemical, Nacalai Tesque, R & D Systems, etc.)

(Example 1: Method of fabricating a tubular body coated with a conductive macromolecule)

An Au nanotube was fabricated by the same method as a known method (Scientific Reports, 9, 6806, 2019.)

(Electroless Au thin membrane plating)

TEPC membranes (it4ip S.A.) were treated in a 1.25 M NaOH solution at 40°C for 20 minutes. After washing the membranes with water, the membranes were immersed in a 1.25 M $SnCl_2$ solution at 25°C for 10 minutes, then washed with water. An $Sn^{2+}$ coated TEPC membrane was immersed in a 1.25 M$PdCl_2$ solution at 25°C for 10 minutes to form a $Sn^{4+}$ Pd metal catalyst on the membrane surface. This metal formation cycle was repeated 1 to 4 times. The catalyst coated TEPC membrane was immersed in an electroless gold plating solution comprising 200 ml/L NC gold PDII (NC gold II; Kojima Chemical) and 20 ml/L sodium disulfitoaurate (I) at 40°C for 24 hours. After washing the Au/TEPC membrane with water, this was dried in a vacuum chamber.

(Au nanostraw fabrication by wet etching and dry etching)

An Au/TEPC membrane was floated for 4 minutes on aqua regia (ITO-02; Kanto Chemical Co., Inc.), and the top surface of the Au nanolayer on the Au/TEPC membrane was etched. The etched Au/TEPC membrane was washed with distilled water and then dried in a vacuum chamber. In order to fabricate an Au nanostraw, the TEPC surface on the Au/TEPC membrane was etched by oxygen-based reactive ion etching. The height of an Au nanostraw was able to be controlled by changing the etching time. After etching, a scanning electron microscope (SEM) image of the Au nanostraw was obtained using HITACH SEM S-3400N and HITACH SEM software, and the outer and inner diameters of over 100 Au nanostraws were measured.

(Example 2: Coating with conductive macromolecule monomer)

A technology that is known (ACS Macro Lett. 2012, 1, 3, 400-403) was used as a method of electropolymerization of a conductive macromolecule monomer (PEDOT) on an Au nanotube. In this regard, the surface area of Au is about 88.7 $cm^2$. PEDOT is electropolymerized on Au by applying a constant voltage of 1 V in an aqueous monomer solution (0.05 M PEDOT, 0.1 M $LiClO_4$). This is controlled by the polymerization time in this Example. PEDOT membranes were fabricated in 3 min (amount of polymerization: about 1.75 C), 5 min (amount of polymerization: about 2.49 C), and 7 min (amount of polymerization: about 3.05 C).

(Example 3: System for penetrating a composite nanotube (conductive macromolecule and gold) into a cell)

Generally, bright field observation or a confocal laser microscope while allowing transmission of light is used for observation of cells. Meanwhile, cell stamping of the Applicant blocks transmission of light, so that observation was not possible (Figure **1**). Introduction efficiency (85.7%) and cell viability rate (94.0%) were obtained in known results up to this point (Scientific Reports, 9, 6806, 2019.) by introducing calcein molecules into a cell while using a measurement equipment capable of measuring the needle height with a precision of 1 $\mu$m (Figure 2a). However, in view of the height of adhering cells being about 1 $\mu$m, stamping methods using a conventional measurement equipment had an issue in that damage to the cell is induced by inserting too far, and insufficient insertion makes it difficult to introduce a substance. In this regard, introduction efficiency (99.9%) and cell viability rate (99.5%) were successfully obtained by incorporating differential interference contrast (DIC) (optional mirror unit known as Olympus U-MDIC3), which can insert a needle while observing cells from below (Figure 2b).

(Example 4: Change in cell viability rate by penetration into a cell using a gold nanotube and composite nanotube (conductive macromolecule and gold))

Use of a composition nanotube can extend the period of insertion into a cell. When a gold nanotube was inserted into a cell in a system for intracellular penetration described above, it was found that the viability rate of cells decreased with time and reached about 80% after 30 minutes (Figure **3**). Meanwhile, when a composite nanotube was inserted, the decrease in the cell viability rate was more gradual, exhibiting 93.8% after 30 minutes. The reason for this is understood to be coating with a conductive macromolecule, which was able to prevent outflow from within a cell.

(Example 5: Controlling introduction of calcein molecules into a cell by applying a voltage to a composite nanotube)

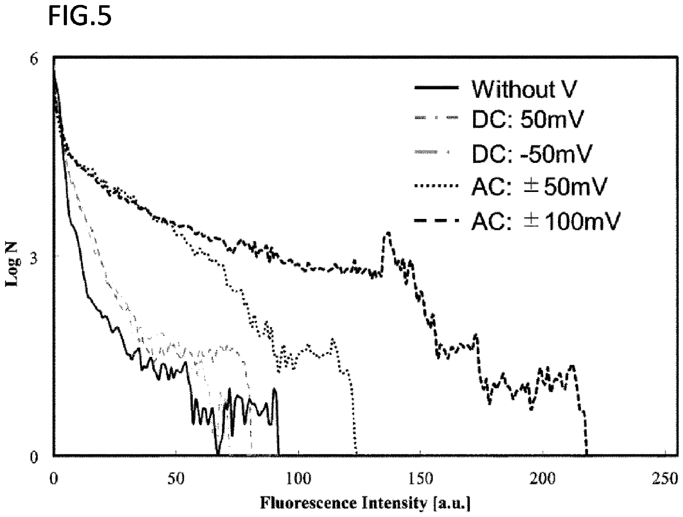

A stamp combining a composite nanotube (5 min polymerization) and a model substance (low molecular weight calcein (622.55 g/mol)) was inserted into a human derived cancer cell (HeLa), and introduction of calcein molecules into a cell by turning a voltage On/Off was observed under a fluorescence microscope. When a voltage was turned off (Figure **4a**), calcein molecules were not introduced into a cell, and the viability rate exhibited a high value of 99.02% after 10 minutes. Meanwhile, calcein molecules were introduced into a cell by applying an AC voltage ($\pm$ 50 mV) to a composite nanotube, and stained cells were able to be confirmed (Figure **4b**). Introduction efficiency of nearly 100%, as well as a cell viability rate of a high value of 98.78% were exhibited. It was also found that when an AC voltage of the cell membrane potential (approximately 70 mV) or greater $\pm$ 100 mV was applied, introduction efficiency of nearly 100% was exhibited while the viability rate maintained at a high value of 84.10%, albeit with a slight decrease, was exhibited (Figure **4c**). Interestingly, it was found that when a DC voltage (50 mV and -50 mV) was applied to a composite nanotube, the amount of calcein introduction dramatically decreased, while the viability rate was maintained at 98.7% (Figures **4d** and **e**). As shown in Figure **5,** more increase in fluorescence intensity was confirmed for higher AC voltage. Meanwhile with DC voltages, the amount of calcein introduced into a cell did not increase significantly, despite applying the same value of voltage as AC voltage. Since it has confirmed that the inner diameter of a composite tube changes from applying a DC voltage, an increase in the flow rate due to the change in the inner diameter can be deemed as the cause of an increase in the amount of calcein introduced. Meanwhile, when an AC voltage is applied, a PEDOT conductive macromolecule repeats volume expansion and contraction. Thus, it is understood that such a change in volume is generated within a composite nanotube and serves a role of a pump or the like to improve the introduction of calcein molecules.

(Example 6: Controlling introduction of a protein into a cell by applying a voltage to a composite nanotube)

Introduction of a protein into a cell is expected as a technology that can modify the function without gene transfer such as direction induction (direction reprogramming) from a skin cell into a pluripotent hematopoietic stem cells in addition to induction (reprogramming) from skin cells into induced pluripotent stem (iPS) cells. However, it is extremely challenging to introduce a large molecule such as a protein into a cell coated with a lipid bimolecular membrane. In this regard, it was confirmed that a protein is efficiently introduced into a cell by utilizing the present invention that improves introduction of a substance via a pumping effect associated with application of an AC voltage and a composite nanotube.

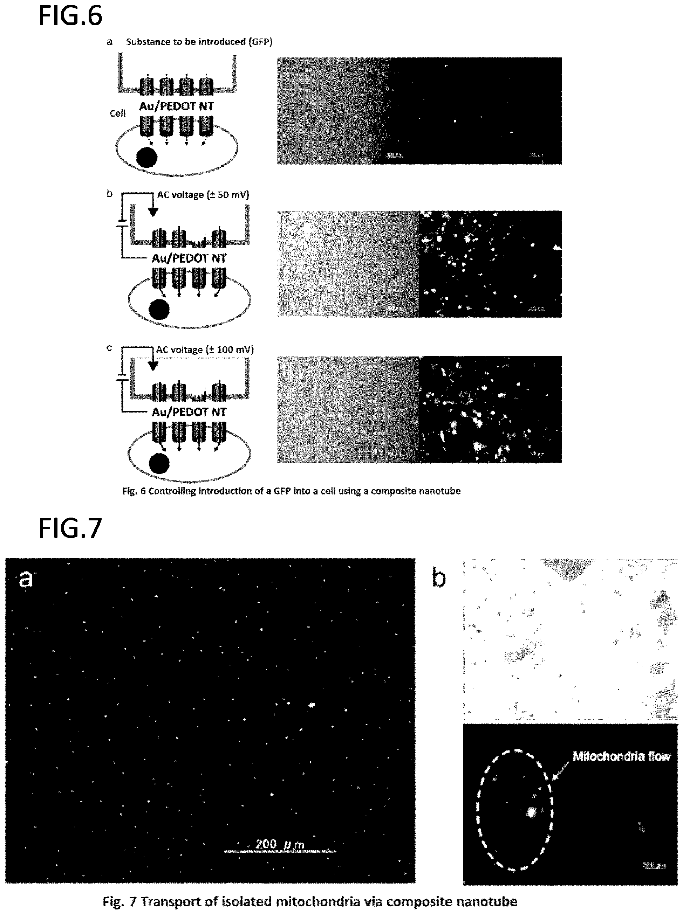

In this Example, the same experiment as Example 5 was conducted after changing the model substance from a low molecular weight calcein to a macromolecule green fluorescence protein (GFP, molecular weight: 27 kDa). When the voltage was turned off (Figure **6a**), a GFP protein was not introduced into a cell, but it was confirmed that a GFP was introduced into a cell by applying an AC voltage ($\pm$ 50 mV, $\pm$ 100 mV). It was also confirmed that the amount introduced was increased by increasing the voltage.

(Example 7: Transport of isolated mitochondria via a composite nanotube)

The composite nanotube can also transport an even larger molecule (organelle mitochondria). A calcein labeled isolated mitochondrion solution prepared from staining mitochondria isolated from a swine heart with a calcein dye, when applied onto a substrate, adsorbs to the substrate as shown in Figure **7a,** which can be confirmed through fluorescence observation. While mitochondria have a complex shape from repeating division and fusion in a cell, isolated mitochondria are spherical with a diameter of about 500 to 1000 nm. When it was checked whether mitochondria can be transported in a nanotube by combining the mitochondrion solution and the composite nanotube, it was confirmed that mitochondria were transported via a composite nanotube thin membrane and adsorbed onto the substrate as shown in Figure **7b.**

(Example 8: Evaluation of structure of composite nanotube and ion permeation)

(Structural analysis by scanning electron microscope (SEM))

The top and bottom surfaces of the composite nanotube thin membrane fabricated above were observed under an SEM to check the polymerization of PEDOT over time (Figure **8a**). An SEM image of PEDOT coating on the bottom surface (needle side) confirmed an increase in membrane thickness over time (Figure **8c**). Furthermore, when the top surface (flat side without a needle) was observed, it was revealed that the inner diameters coated with PEDOT are all the same independent of time (Figures **8b** and **c**). Specifically, it was confirmed that coating of PEDOT on an Au nanotube thin membrane is a structure that is asymmetric between the top and bottom surfaces (Figure **8d**).

(Example 9: Permeability of a substance calcein through a gold nanotube and composite nanotube thin membranes)

The amount of calcein substance that passes through a gold nanotube and a composite nanotube thin membrane was quantitatively evaluated by the same method as a known method (Scientific Reports, 9, 6806, 2019.) The flow rate J of a substance can be expressed by the following equation:

[Numeral 1]

$$J = DC \left( {\pi r^2 n} \middle/ {\pi R^2} \right) / l$$

wherein D is a diffusion coefficient, C is the calcein concentration, $\pi r^2$ is the inside area of an Au nanostraw, n is the number of Au naonstraws, $\pi R^2$ is the area of the membrane, and l is the thickness of the membrane.

As shown in Figure **9,** it was found that the flow rate was 86.9 nmol after 20 minutes when a gold nanotube (0 min coating) was used, whereas the flow rate decreased with a composite nanotube coated with PEDOT. It was found that the flow rate of calcein exhibited a value that was nearly zero with a composite nanotube coated with PEDOT for 5 minutes or longer. It can be understood that this is primarily due to a smaller inner diameter (r) through coating of PEDOT. Since a dried composite nanotube is observed under an SEM in vacuum, PEDOT macromolecules are contracted. In view of a PEDOT membrane that is swollen from containing moisture in a solution, the actual inner diameter of a composite nanotube would be smaller.

(Example 10: Controlling On/Off of permeation of calcein by applying a voltage to a composite nanotube)

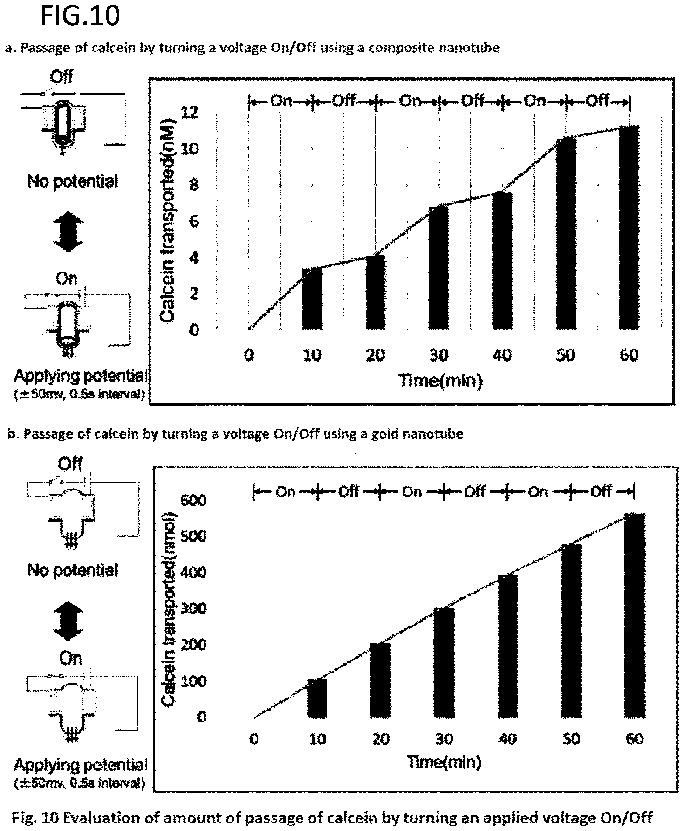

It is known that the volume of a PEDOT conductive macromolecule changes by applying a voltage (Journal of American Chemical Society, 132, 13174-13175, 2010.) In this regard, a test was conducted to check whether calcein permeation changes by applying a voltage to a composite nanotube (Figure **10a**). It was found as a result that the amount of permeation can be controlled by turning the voltage On/Off. Meanwhile, calcein permeation could not be stopped even after applying the same voltage to only a gold nanotube (Figure **10b**). This revealed for the first time that the change in volume of a PEDOT macromolecule contributes to On/Off control.

Furthermore, whether the shape of a PEDOT macromolecule is important can be checked by using a nanotube coated with PEDOT on both surfaces of an Au thin membrane without a needle.

(Example 11: Experiment for "recovery" from a cell)

This Example demonstrates whether recovery from a cell is possible.

In this Example, cells are stained by introducing calcein AM into cells in the same manner as Example 5. A composite nanoneedle of a stamp comprising the same solution as the stamp used in introducing calcein AM, other than not containing calcein AM, in a storage unit is inserted into a cell to confirm that there is no recovery without application of voltage and recovery of low molecular weight calcein is promoted by applying a voltage to a composite nanotube.

Conditions under which recovery changes depending on the size of voltage applied and type of voltage are studied.

It is demonstrated that recovery of not only small molecules but also macromolecules (GFP) is possible.

(Example 12: Demonstration example for "nucleic acid" (oligo DNA) through application of a voltage to a composite nanotube)

This Example demonstrates whether a nucleic acid (oligo DNA) can be moved in and out of a cell.

Commercially available florescent dye labeled oligo DNA was placed in a composite nanotube device, and an intracellular introduction test and a recovery test from a cell were conducted in the same manner as Examples 5 and 11. After inserting a composite nanoneedle into a cell, the tests check that there is no introduction or recovery without application of a voltage and introduction and recover of a nucleic acid are promoted by applying a voltage to a composite nanotube.

Conditions under which introduction and recovery change depending on the size of voltage applied and type of voltage are studied.

(Example 12: Demonstration example for "peptide" or "protein" (GFP) by applying a voltage to a composite nanotube)

This Example demonstrates whether a "peptide" or "protein" (GFP) can be moved in and out of a cell.

Commercially available florescent dye-labeled peptide or protein was placed in a composite nanotube device, and an intracellular introduction test and a recovery test from a cell were conducted in the same manner as Examples 5 and 11. After inserting a composite nanoneedle into a cell, the tests check that there is no introduction or recovery without application of a voltage and introduction and recover of a substance are promoted by applying a voltage to a composite nanotube.

Conditions under which introduction and recovery change depending on the size of voltage applied and type of voltage are studied.

(Example 13: Demonstration example for "organelle" (mitochondria) by applying a voltage to a composite nanotube)

This Example demonstrates whether an "organelle" (mitochondria) can be moved in and out of a cell.

Mitochondria isolated from a cell were placed in a composite nanotube device, and an intracellular introduction test and a recovery test from a cell were conducted in the same manner as Examples 5 and 11. After inserting a composite nanoneedle into a cell, the tests check that there is no introduction or recovery without application of a voltage and introduction and recover of mitochondria are promoted by applying a voltage to a composite nanotube.

Conditions under which introduction and recovery change depending on the size of voltage applied and type of voltage are studied.

(Example 14: Test for extracting an intracellular substance using a composite nanotube)

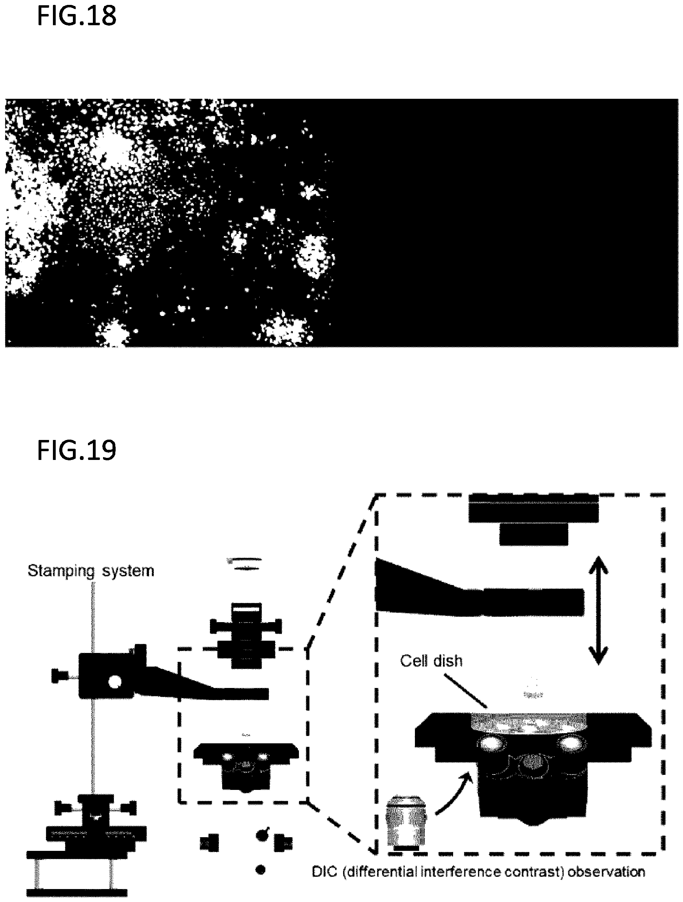

In this Example, an attempt was made to extract an intracellular substance by using a composite nanotube. First, HeLa cells were cultured for 30 minutes in a culture comprising calcein AM (concentration 25 μm), though which cell membrane permeable calcein AM was taken up into a cell and reacted with esterase in the cell to be membrane impermeable calcein. As a result, calcein fluorescent dye was able to be retained in a cell (Figure **17a**). Since calcein is a fluorescent dye, fluorescence of 515 nm is emitted by irradiating an excitation light of 490 nm. Thus, the intracellular calcein concentration can be evaluated by measuring the fluorescence intensity. When a composite nanotube is inserted therein for about 30 seconds, calcein dyes contained in the cell can be extracted (Figure **17b**). As can be understood from Figure **17b,** fluorescence intensity is reduced only in a region where a composite nanotube is inserted. After extraction, cells can take up calcein dyes (Figure **17c**). A viability rate of 99.56% (viable cell count: 3600, dead cell count: 16) was successfully achieved in the extraction region (insertion region) as a result of staining with PI for measuring dead cells (Figure **18**). The amount of extraction can be controlled by changing the penetration time or inner diameter of a composite nanotube.

(Example 15: Test for introducing a microsphere into a cell)

Figure **19** shows the configuration of an experiment for introducing a microsphere into a cell by a stamping system. A stamping system with a composite nanotube incorporated therein was introduced into a cell while observing, in real-time, with a Differential Interference Contrast (DIC) system of an inverted fluorescence microscope. The composite nanotube membrane stamp used in the experiment had a membrane diameter: 8 mm, nanotube diameter: 1 $\mu$m, nanotube density: 2.2E7, nanotube length from membrane: 2.4 $\mu$m, and full length of nanotube: 24 $\mu$m. A test was conducted by combining the stamp with a mixture solution (calcein: 1.6 mM, fluorescent dye labeled microsphere: 1%, volume: 300 $\mu$l) and puncturing a cell with a stamp for 10 minutes to introduce a microsphere into a cell. Since calcein is a cell membrane impermeable small molecule that can be readily introduced and exhibits yellowish green fluorescence, calcein was used to confirm that a cell was punctured by a nanotube.

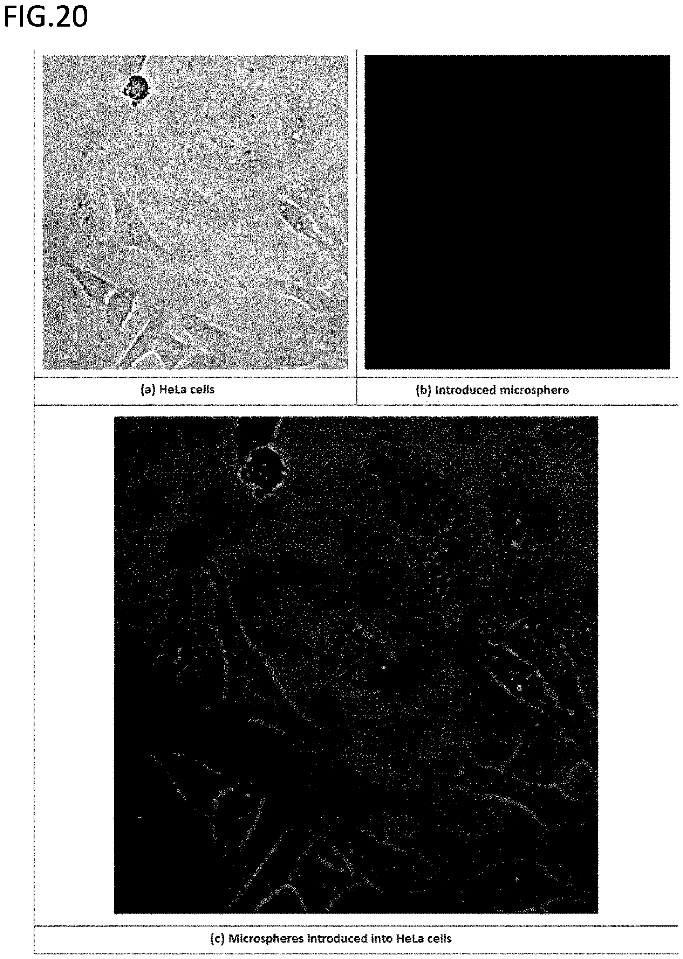

The results for microspheres introduced into a cell by stamping are shown in Figure 20. (a) is an image of HeLa cells with a microsphere introduced therein, (b) is a fluorescence image of microspheres introduced into cells, and (c) is an image from superimposing the images of (a) and (b). It was found from the results of Figure **20** that microspheres are introduced uniformly into HeLa cells.

(Example 16: Promotion of transport of a substance using a microtube)

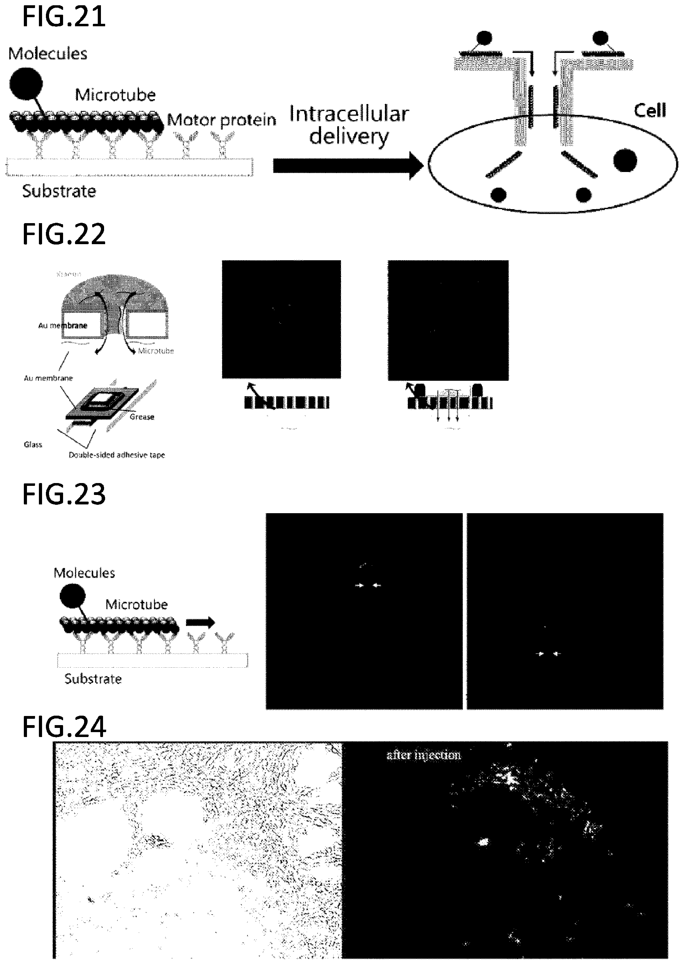

This Example for promoting transport of a substance into cells was conducted by combining a mechanism of a biomolecular motor and a composite nanotube membrane (Figure **21**). Specifically, a kinesin molecular motor was modified and attached to a microtube on a gold membrane. In the presence of ATP in a solution, the chemical energy of the ATP is converted to mechanical energy so that the microtube moves on the substrate. The rate of movement of a molecular motor is generally known to be 0.4 to 4.0 $\mu$m/s. A rate of 0.4 to 2.0 $\mu$m/s was exhibited on a membrane developed by the inventors (Figure **22**). The movement rate can be adjusted by changing the convexity or concavity of the substrate, modification method, or ATP concentration. It was confirmed that a microtube passed through a membrane via a nanotube (Figure **6**). When a microsphere with a diameter of about 30 nm was chemically attached to a microtube (biotin-avidin reaction), the microsphere was successfully transported while maintaining the rate of 1.86 $\mu$m/s (Figure **23**). A microsphere without a chemical modification, when floated in a solution, would not move in a certain direction by Brownian motion (resulting in random movement). The rate of gradual settling due Stoke's law is very low at $1.1 \times 10^{-5}$ $\mu$m/s when assuming a polystyrene microparticle (100 nm diameter).

(Example 17: Introduction of Oct4 protein into a cell)

Oct4 is a transcription factor known to be able to control induction of differentiation of cells by controlling the amount of expression thereof. This Example shows the possibility of inducing differentiation of cells by introducing a functional protein Oct4 into a cell. Since an Oct4 protein is colorless, it is difficult to evaluate whether it was introduced into a cell. In this regard, an Oct4 protein was modified with a green fluorescent protein GFP so that green fluorescence is emitted in a cell to confirm that the Oct4 protein has been introduced into a cell (Figure **24**).

As described above, the present invention is exemplified by the use of its preferred embodiments. It is understood that the scope of the present invention should be interpreted solely based on the Claims. The present application claims priority to Japanese Patent Application No. 2020-41164 (filed on March 10, 2020). It is understood that the entire content thereof is incorporated herein by reference. It is also understood that any patent, any patent application, and any references cited herein should be incorporated herein by reference in the same manner as the contents are specifically described herein.

[Industrial Applicability]

The present disclosure can deliver and recover a substance such as a reagent into/from a cell, and can be applied in not only the manufacturing industries such as the cosmetics industry, cosmetology, health, bioengineering, pharmaceuticals, seasoning, and fragrance, but also various industries.

[Reference Signs List]

Handle of a stamp **1**
(Substance) storage unit **2**
(Membranous) substrate **3**
Substance **4**
Penetrating tubular body **5**

**Claims**

1. A tubular body coated with a conductive macromolecule for use in introducing a substance into a cell and/or recovering a substance from a cell.

2. Use of a tubular body coated with a conductive macromolecule for introducing a substance into a cell and/or recovering a substance from a cell.

3. The tubular body or use of claim 1 or 2, wherein the tubular body is made of a conductor.

4. The tubular body or use of claim 3, wherein the conductor comprises a metal.

5. The tubular body or use of claim 4, wherein the metal comprises a metal that can be applied by electroless plating.

6. The tubular body or use of claim 4 or 5, wherein the metal comprises at least one metal selected from the group consisting of gold, platinum, silver, and nickel, and alloys thereof.

7. The tubular body or use of any one of claims 1 to 4, wherein the conductive macromolecule has a property of expanding and contracting when a voltage is applied.

8. The tubular body or use of claim 7, wherein an absolute value of the voltage is 500 mV or less.

9. The tubular body or use of any one of claims 7 to 8, wherein an absolute value of the voltage is 100 mV or less.

10. The tubular body or use of any one of claims 7 to 9, wherein an absolute value of the voltage is 50 mV or less.

11. The tubular body or use of any one of claims 7 to 10, wherein the voltage is an AC voltage.

12. The tubular body or use of any one of claims 1 to 11, wherein the conductive macromolecule comprises at least one macromolecule selected from the group consisting of poly(3,4-ethylene dioxythiophene) (PEDOT), polythiophene, polyacetylene, polyaniline, polypyrrole, and combinations thereof.

13. The tubular body or use of any one of claims 1 to 12, wherein the tubular body has an inner diameter of 2 $\mu$m or less.

14. The tubular body or use of any one of claims 1 to 13, wherein the tubular body has an outer diameter of 4 $\mu$m or less.

15. The tubular body or use of any one of claims 1 to 14, wherein the tubular body has a length of 5 $\mu$m or greater and 50 $\mu$m or less.

16. The tubular body or use of any one of claims 1 to 15, wherein the substance has an arithmetic mean diameter of 1 nm or greater and 2 $\mu$m or less.

17. The tubular body or use of any one of claims 1 to 16, wherein the substance comprises at least one selected from the group consisting of a low molecular weight compound, a nucleic acid, a peptide, a protein, and an organelle.

18. A device for use in introducing a substance into a cell and/or recovering a substance from a cell, comprising a tubular body coated with a conductive macromolecule, a substrate, and an electrode, wherein the tubular body is disposed on the substrate.

19. The device of claim 18, wherein the device comprises a plurality of the tubular bodies.

20. The device of claim 18 or 19, wherein the device further comprises a storage unit for storing the substance.

21. The device of any one of claims 18 to 20, wherein the substrate comprises polycarbonate and/or polyethylene terephthalate.

22. The device of any one of claims 20 to 21, wherein the storage unit has a structure, which has an inside that is a void, is directly connected to the substrate, and has a tube through which an electrode can be inserted into the void.

23. The device of any one of claims 18 to 22, wherein the storage unit is comprised of at least one material selected from the group consisting of glass, ceramic, macromolecule, and metal.

24. The device of any one of claims 18 to 23, wherein the tubular body further comprises a feature of any one of claims 1 to 17.

25. The device of any one of claims 20 to 24, wherein the storage unit comprises the substance.

26. The device of any one of claims 18 to 25, wherein the electrode is provided with two poles for applying a voltage to the conductive macromolecule and a storage unit.

27. A system for use in introducing a substance into a cell and/or recovering a substance from a cell, comprising:

the device of any one of claims 18 to 26; and
a voltage supplying unit for supplying a voltage.

28. The system of claim 27, further comprising a container comprising the cell.

29. The system of claim 27 or 28, further comprising an observation unit for differential interference contrast observation of the cell in a container from below.

30. The system of claim 29, wherein the observation unit is materialized by a differential interference contrast prism.

31. The system of any one of claims 27 to 30, further comprising a display unit for displaying an image from the observation unit.

32. The system of any one of claims 27 to 31, wherein insertion of a tubular body contained in the device into the cell can be adjusted in units of 0.1 $\mu$m.

33. A method for introducing a substance into a cell and/or recovering a substance from a cell, comprising the steps of:

(a) providing the device of any one of claims 18 to 26 or the system of any one of claims 27 to 32; and
(b) applying a voltage to the tubular body over a desired period of time.

34. A method of fabricating a device comprising a tubular body coated with a conductive macromolecule and a substrate, comprising the step of:
securing a membrane of the conductive macromolecule to a substrate on which the tubular body is disposed at one or more points to coat the tubular body.

35. The method of claim 34, wherein the step of coating comprises the step of securing the membrane of the conductive macromolecule at two or more points to coat the tubular body.

36. The method of claim 34 or 35, wherein the step of coating is achieved by providing working electrodes that contact the substrate at two or more points.

37. The method of any one of claims 34 to 36, wherein the substrate on which the tubular body is disposed is fabricated by a process comprising the step of electroless plating of a metal on track-etched polycarbonate and the step of etching a metal plated surface.

38. The method of any one of claims 34 to 37, wherein an inner diameter of the tubular body is 0.01 $\mu$m to 2 $\mu$m, and an outer diameter is 0.1 $\mu$m to 5 $\mu$m.

39. A method of activating a cell, comprising the step of introducing a mitochondrion into the cell by using the device of any one of claims 18 to 26 or the system of any one of claims 27 to 32.

40. A method of controlling a cellular function, comprising the step of introducing a gene product (protein) into the cell by using the device of any one of claims 18 to 26 or the system of any one of claims 27 to 32.

41. The method of claim 40, wherein the gene product comprises a gene product of Oct4.

42. A tubular body coated with a conductive macromolecule for use in introducing a substance into a cell and/or recovering a substance from a cell, wherein the conductive macromolecule comprises kinesin.

43. Use of a tubular body coated with a conductive macromolecule comprising kinesin for introducing a substance into a cell and/or recovering a substance from a cell.

44. A device for use in introducing a substance into a cell and/or recovering a substance from a cell, comprising a tubular body coated with a conductive macromolecule comprising kinesin, a substrate, and an electrode, wherein the tubular body is disposed on the substrate.

45. A system for use in introducing a substance into a cell and/or recovering a substance from a cell, comprising:

   the device of claims 44; and
   a voltage supplying unit for supplying a voltage.

46. A method for introducing a substance into a cell and/or recovering a substance from a cell, comprising the steps of:

   (a) providing the device of claim 44 or the system of claim 45;
   (b) providing a microtube with a substance attached thereto; and
   (c) applying a voltage to the tubular body over a desired period of time.

47. A method of fabricating a tubular body coated with a conductive macromolecule comprising kinesin, comprising the step of attaching kinesin to the tubular body.

48. A stamping system for use in inserting a composite nanotube into a cell, comprising:

   a stamp comprising a tubular body coated with a conductive macromolecule;
   an electrode; and
   a voltage supplying unit for supplying a voltage.

49. A penetration observation system for use in inserting a composite nanotube into a cell, comprising:

   the device of any one of claims 18 to 26 and 44;
   a voltage supplying unit for supplying a voltage; and
   a microscope.

50. A composite nanotube thin membrane and stamp kit for delivering a substance into a cell or extracting a substance from a cell, comprising:

   a composite nanotube thin membrane comprising a tubular body coated with a conductive macromolecule; and
   a stamp.

51. A method of evaluating cell activity, comprising the step of:
measuring a quantity of a marker substance within a cell by using a tubular body coated with a conductive macromolecule.

52. The method of claim 50, further comprising the step of introducing a mitochondrion into a cell.

53. A method of introducing a cell using a microsphere, comprising the steps of:

   (] providing the device of any one of claims 18 to 26 and 44, or the system of any one of claims 27 to 32 and 45;
   (b) providing a microsphere; and
   (c) applying a voltage to the tubular body over a desired period of time.

54. The method of claim 53, further comprising the step of attaching a substance to the microsphere.

**55.** A method of extracting an intracellular substance, comprising the steps of:

(a) providing the device of any one of claims 18 to 26 and 44, or the system of any one of claims 27 to 32 and 45; and
(b) applying a voltage to the tubular body over a desired period of time.

**56.** A method of cell reprogramming, comprising the steps of:

(a) providing the device of any one of claims 18 to 26 and 44, or the system of any one of claims 27 to 32 and 45;
(b) providing a gene or a gene product; and
(c) applying a voltage to the tubular body over a desired period of time.

**57.** A method of introducing a functional protein into a cell to modify a phenotype of the cell, comprising the steps of:

(a) providing the device of any one of claims 18 to 26 and 44, or the system of any one of claims 27 to 32 and 45;
(b) providing a functional protein; and
(c) applying a voltage to the tubular body over a desired period of time.

EP 4 119 650 A1

# FIG.1

Fig. 1 System for penetrating a nanotube stamp into a cell, which incorporates DIC observation

# FIG.2

**a. Stamping of cells without DIC observation**

$$\text{Introduction efficiency} = \frac{\text{Viable or dead cell count}}{\text{Total cell count}} = 85.7\%$$

$$\text{Viability rate} = \frac{\text{Viable cell count}}{\text{Viable or dead cell count}} = 94.0\%$$

**b. Stamping of cells with addition of DIC observation**

$$\text{Introduction efficiency} = \frac{\text{Viable or dead cell count}}{\text{Total cell count}} = 99.9\%$$

$$\text{Viability rate} = \frac{\text{Viable cell count}}{\text{Viable or dead cell count}} = 99.5\%$$

Fig. 2 Substance introduction efficiency and cell viability rate with or without DIC observation mechanism

FIG.3

Fig. 3 Cell viability rate: Insertion of gold nanotube and composite nanotube into cell

# FIG.4

Fig. 4 Controlling introduction of a fluorescent substance calcein into a cell using a composite nanotube

FIG.5

# FIG.6

a   Substance to be introduced (GFP)

Au/PEDOT NT

Cell

b   AC voltage (± 50 mV)

Au/PEDOT NT

c   AC voltage (± 100 mV)

Au/PEDOT NT

Fig. 6 Controlling introduction of a GFP into a cell using a composite nanotube

# FIG.7

a

200 μm

b

Mitochondria flow

Fig. 7 Transport of isolated mitochondria via composite nanotube

# FIG.8

a  Bottom surface

b  Top surface

c

d  Top surface

Bottom surface

Fig. 8 Structural analysis of PEDOT electropolymerized composite nanotube thin membrane

# FIG.9

Fig. 9 Measurement of the concentration of calcein passing through a gold nanotube (0 min) and composite nanotube thin membrane

# FIG.10

**a. Passage of calcein by turning a voltage On/Off using a composite nanotube**

**b. Passage of calcein by turning a voltage On/Off using a gold nanotube**

Fig. 10 Evaluation of amount of passage of calcein by turning an applied voltage On/Off

# FIG.11

(a)

| | Electroless Au plating | | Etching of top surface | |
|---|---|---|---|---|

(b) **Face up**

Face down

(c) **Face up**

Face down

(d) **Face up**

Face down

(e) RIE 0 min

TEPC

(f) 10 min

(g) 20 min

(h) 30 min

# FIG.12

(a)

| | Sensitization | Activation | Displacement | Electroless deposition |
|---|---|---|---|---|

SnCl₂

Cycle

# FIG.13

a  Substance to be introduced

Application of minute voltage

Application of minute voltage

**Au/PEDOT NT**

Cell

**Au/PEDOT NT**

**Au/PEDOT NT**

Fig. 13 Outline of technology for introducing and extracting a substance into a cell via a composite nanotube (conductive macromolecule and gold)

## FIG.14

| | Chemical | | Physical | | |
|---|---|---|---|---|---|
| | Liposome | Viral vector | Electroporation | Microprocessed nanoneedle | |
| | | | | Single nanoneedle | Plurality of nanoneedles |
| Endocytosis | Required | | Not required | Not required | Not required |
| Introduction efficiency | High | High | Low | High | High |
| Extraction efficiency | Low | Low | Low | High | High |
| Toxicity | Chemical | Biological | None | None | None |
| Throughput | High | High | Low | Low | High |
| Operation | Simple | Simple | Simple | Complex | Simple |

Fig. 14 Classification and superiority of the method of the present disclosure and other methods

## FIG.15

# FIG.16

# FIG.17

FIG.18

FIG.19

Stamping system

Cell dish

DIC (differential interference contrast) observation

# FIG.20

| | |
|---|---|
| (a) HeLa cells | (b) Introduced microsphere |

(c) Microspheres introduced into HeLa cells

# FIG.21

Molecules

Microtube

Motor protein

Substrate

Intracellular delivery

Cell

# FIG.22

# FIG.23

Molecules

Microtube

Substrate

# FIG.24

after injection

# FIG.25

a **Damage free isolated mitochondria**

b **Introduction of isolated mitochondria into skin cells**

c **Cell activity evaluation (improvement in senescence)**

Highly active mitochondria

Senescence marker substance

Artificial nanotube

Artificial nanotube

Low activity mitochondria

Skin cell

Improvement in function

Regenerative medicine "cell therapy", cosmetology field

# FIG.26

## Settling rate VS Biomolecular motor rate

Microsphere : Φ100 nm

Settling according to Stoke's Law

[Resistance]
[Buoyance]
[Gravity]

Settling rate
$$V = \frac{2g \times r^2 \times (\rho - \rho_{water})}{9\eta}$$

V: Settling rate $(cm/s)$

g: Gravitational acceleration $(9.8 \times 10^2 cm/s)$

r: radius of particle (cm) $(cm)$

$\rho$: density of particle (g/cm³)

$\rho_{water}$: density of water (g/cm³)

$\eta$: viscosity coefficient $(\frac{g}{cm} \cdot s)$

**Settling rate** : $1.1 \times 10^{-5}$ µm/s

*polystyrene microparticle (100 nm), assuming water temperature of 30 degrees

Microsphere : Φ100 nm

**Biomolecular motor**

Kinesin    Microtubule

Faster

Gold thin film

Glass substrate

Microtubule

Kinesin

Gliding direction

Micro-structure

**Biomolecular motor rate** : 0.4 - 4.0 µm/s

*not the rate when microparticles are modified

**INTERNATIONAL SEARCH REPORT**

| International application No. |
| --- |
| PCT/JP2021/009347 |

**A. CLASSIFICATION OF SUBJECT MATTER**

C12M 1/00(2006.01)i; C12M 1/26(2006.01)i; C12N 15/87(2006.01)i; C12Q 1/24(2006.01)i
FI: C12M1/00 A.; C12M1/26; C12N15/87 Z; C12Q1/24

According to International Patent Classification (IPC) or to both national classification and IPC

**B. FIELDS SEARCHED**

Minimum documentation searched (classification system followed by classification symbols)
C12M1/00-3/10; C12N15/00-15/90; C12Q1/00-3/00

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); Japio-GPG/FX

**C. DOCUMENTS CONSIDERED TO BE RELEVANT**

| Category* | Citation of document, with indication, where appropriate, of the relevant passages | Relevant to claim No. |
| --- | --- | --- |
| X | US 2008/0227168 A1 (KMECKO, T.) 18 September 2008 (2008-09-18) claims, paragraphs [0008]-[0013], [0025]-[0085] | 1-3, 7-17 |
| A | claims, paragraphs [0008]-[0013], [0025]-[0085] | 4-6, 18-57 |
| X | JP 2010-501260 A (THE REGENTS OF THE UNIVERSITY OF MICHIGAN) 21 January 2010 (2010-01-21) claims, paragraphs [0016]-[0063] | 1-2, 7-19, 24, 27-28, 33-36, 38-41, 49, 51-57 |
| A | claims, paragraphs [0016]-[0063] | 3-6, 20-23, 25-26, 29-32, 37, 42-48, 50 |
| A | ZHANG, B. et al., "Nanostraw membrane stamping for direct delivery of molecules into adhesive cells", Scientific Reports, 2019, vol. 9, 6806 (pp. 1-8), abstract, results, fig. 1-4 | 1-57 |

☒ Further documents are listed in the continuation of Box C.  ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 17 May 2021 (17.05.2021) | 25 May 2021 (25.05.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2021/009347 |

C (Continuation).    DOCUMENTS CONSIDERED TO BE RELEVANT

| Category* | Citation of document, with indication, where appropriate, of the relevant passages | Relevant to claim No. |
|---|---|---|
| A | IDO, Y. et al., "Conducting Polymer Microelectrodes Anchored to Hydrogel Films", ACS Macro Lett., 2012, vol. 1, pp. 400-403, abstract, page 400, left column, paragraph [0002] to page 402, right column, paragraph [0001], fig. 1-4 | 1-57 |
| A | JP 2019-528782 A (THE BOARD OF TRUSTEES OF THE LELAND STANFORD JUNIOR UNIVERSITY) 17 October 2019 (2019-10-17) claims | 1-57 |
| A | JP 2019-517273 A (THE BOARD OF TRUSTEES OF THE LELAND STANFORD JUNIOR UNIVERSITY) 24 June 2019 (2019-06-24) claims | 1-57 |
| A | WO 2004/033614 A1 (FUJITSU LTD.) 22 April 2004 (2004-04-22) claims | 1-57 |
| P, A | 小山和洋ほか，人工トンネリングナノチューブを介した高効率な細胞内物質導入システムの開発，第67回応用物理学会春季学術講演会 講演予稿集，10 March 2020, pp. 10-122 (12p-A408-4), "2. Experimental procedure and results", (OYAMA, Kazuhiro et al., "High-efficiency molecular delivery into adhesive cells via artificial tunneling nanotubes", Lecture preprints of the 67th JSAP Spring Meeting 2020) | 1-57 |

Form PCT/ISA/210 (continuation of second sheet) (January 2015)

**INTERNATIONAL SEARCH REPORT**
Information on patent family members

International application No.

PCT/JP2021/009347

| Patent Documents referred in the Report | Publication Date | Patent Family | Publication Date |
|---|---|---|---|
| US 2008/0227168 A1 | 18 Sep. 2008 | (Family: none) | |
| JP 2010-501260 A | 21 Jan. 2010 | WO 2008/085199 A2 claims, paragraphs [0027]-[0074] | |
| JP 2019-528782 A | 17 Oct. 2019 | WO 2018/053020 A1 claims | |
| JP 2019-517273 A | 24 Jun. 2019 | WO 2017/214541 A1 claims | |
| WO 2004/033614 A1 | 22 Apr. 2004 | US 2005/0266568 A1 claims | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019528782 W
- JP 2019517273 W
- JP 2020041164 A

### Non-patent literature cited in the description

- **JJ VAN DERSARL ; AM XU ; NA MELOS.** Nanostraws for direct fluidic intracellular access. *Nano letters,* 2011, vol. 12 (8), 3881-3886
- **A TAY ; N MELOSH.** Nanostructured Materials for Intracellular Cargo Delivery. *Accounts of chemical research,* 2019, vol. 52 (9), 2462-2471
- **Y CAO ; H CHEN ; R QIU ; M HANNA ; E MA ; M HJORT ; A ZHANG ; RS LEWIS ; JC WU ; NA MELOSH.** Universal intracellular biomolecule delivery with precise dosage control. *Science advances,* 2019, vol. 4 (10), eaat8131
- **RUI WEN ; AIHUA ZHANG ; DI LIU ; JIANMING FENG ; JIANG YANG ; DEHUA XIA ; JI WANG ; CHUNWEI LI ; TAO ZHANG ; NING HU.** Intracellular Delivery and Sensing System Based on Electroplated Conductive Nanostraw Arrays. *ACS applied materials & interfaces,* 2019
- **GEN HE ; JIANMING FENG ; AIHUA ZHANG ; LINGFEI ZHOU ; RUI WEN ; JIANGMING WU ; CHENGDUAN YANG ; JIANG YANG ; CHUNWEI LI ; DEMENG CHEN.** Multifunctional Branched Nanostraw-Electroporation Platform for Intracellular Regulation and Monitoring of Circulating Tumor Cells. *Nano letters,* 2019, vol. 19 (10), 7201-7209
- **SIGMA-ALDRICH.** Wako Pure Chemical. Nacalai Tesque, R & D Systems
- *Scientific Reports,* 2019, vol. 9, 6806
- *ACS Macro Lett.,* 2012, vol. 1 (3), 400-403
- *Journal of American Chemical Society,* 2010, vol. 132, 13174-13175